(19) Europäisches Patentamt
European Patent Office
Office européen des brevets

(11) EP 2 300 117 B1

(12) # EUROPÄISCHE PATENTSCHRIFT

(45) Veröffentlichungstag und Bekanntmachung des Hinweises auf die Patenterteilung:
**08.01.2014 Patentblatt 2014/02**

(21) Anmeldenummer: **09772057.7**

(22) Anmeldetag: **28.05.2009**

(51) Int Cl.:
***B01D 11/04*** *(2006.01)* ***C07C 29/86*** *(2006.01)*
***C07C 31/12*** *(2006.01)*

(86) Internationale Anmeldenummer:
**PCT/EP2009/003820**

(87) Internationale Veröffentlichungsnummer:
**WO 2010/000357 (07.01.2010 Gazette 2010/01)**

(54) **VERWENDUNG VON IONISCHEN FLÜSSIGKEITEN MIT TRICYANOMETHIDANIONEN ALS LÖSUNGSMITTEL ZUR EXTRAKTION VON ALKOHOLEN AUS WÄSSRIGEN LÖSUNGEN**

USE OF IONIC LIQUIDS CONTAINING TRICYANOMETHIDE ANIONS AS SOLVENT FOR EXTRACTING ALCOHOLS FROM AQUEOUS SOLUTIONS

UTILISATION DE LIQUIDES IONIQUES CONTENANT DES ANIONS TRICYANOMÉTHANURE COMME SOLVANT POUR L'EXTRACTION D'ALCOOLS À PARTIR DE SOLUTIONS AQUEUSES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **01.07.2008 EP 08011845**

(43) Veröffentlichungstag der Anmeldung:
**30.03.2011 Patentblatt 2011/13**

(73) Patentinhaber: **Merck Patent GmbH**
**64293 Darmstadt (DE)**

(72) Erfinder:
- **PITNER, William-Robert**
  **60599 Frankfurt (DE)**
- **AUST, Emil Ferdinand**
  **55130 Mainz (DE)**
- **SCHULTE, Michael**
  **65474 Bischofsheim (DE)**
- **SCHMID-GROSSMANN, Uschi**
  **64625 Bensheim (DE)**

(56) Entgegenhaltungen:
**WO-A1-2006/021390 GB-A- 2 127 811**
**US-A- 4 445 908 US-A- 4 568 643**

- **J.F. BRENNECKE ET AL: "Liquid Phase Behavior of Ionic Liquids with Water and 1-Octanol and Modeling of 1-Octanol/Water Partition Coefficients" J.CHEM.ENG. DATA, Bd. 52, 10. September 2007 (2007-09-10), Seiten 2462-2467, XP002561599**
- **FADEEV A G ET AL: "OPPORTUNITIES FOR IONIC LIQUIDS IN RECOVERY OF BIOFUELS" CHEMICAL COMMUNICATIONS - CHEMCOM, ROYAL SOCIETY OF CHEMISTRY, GB, Nr. 3, 1. Januar 2001 (2001-01-01), Seite 295/296, XP001120215 ISSN: 1359-7345**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 300 117 B1

## Beschreibung

Gegenstand der Erfindung ist ein Verfahren zur Flüssig-Flüssig-Extraktion von Alkoholen aus wässrigen Lösungen unter Verwendung mindestens einer Ionischen Flüssigkeit enthaltend ein Tricyanomethidanion als Lösungsmittel nach Anspruch 1.

Die Extraktion von Alkoholen aus wässrigen Lösungen gewinnt mehr und mehr an Bedeutung durch intensive Diskussion über "weiße Biotechnologie" bzw. dem Einsatz von Alkoholen als Zusatzstoffe zu konventionellen Treibstoffen. Im Rahmen der intensiven Diskussion von Biotreibstoffen, insbesondere von Bioethanol, zeigt sich, dass Biobutanol, d.h. Butanol welches durch Biomasse hergestellt wird, ebenfalls als potentieller Biotreibstoff von Interesse ist.

Biobutanol hat eine Vielzahl von Vorteilen gegenüber anderen Biotreibstoffen, insbesondere

- kann er aufgrund des geringen Dampfdruckes (5,6 hPa gegenüber 58,5 hPa bei Ethanol) und des geringeren Flammpunktes (36°C gegenüber 12°C für Ethanol) leicht mit konventionellen Treibstoffen gemischt werden,
- zeigt deulich geringeres hygroskopisches Verhalten,
- ist der Energiegehalt dem konventioneller Treibstoffe ähnlicher
- ist beispielsweise weniger korrosiv,
- kann fossile Brennstoffe zu 100% ersetzen ohne notwendige Motormodifizierungen und
- kann mit Biodiesel/Diesel gemischt und in Dieselmotoren genutzt werden.

Die Herstellung von Alkoholen durch Biomasse ist seit langem bekannt. Eine Fermentation, die schon in der ersten Hälfte des zwanzigsten Jahrhunderts industriell eingesetzt wurde, ist die ABE-Fermentation (Aceton-Butanol-Ethanol-Fermentation). Damals wurde der Mikroorganismus *Clostridium acetobutyricum* eingesetzt. Derzeit werden für die Butanolherstellung aus Biomasse weitere Bakterien der Gattung *Clostridium,* wie z.B. *C.beijerinckii, C. saccharoperbutylacetonicum und C.tetanomorphum* eingesetzt. Darüber hinaus werden neue Mikroorganismen, insbesondere Bakterien und Hefen, entwickelt, die einen höheren Butanol-Anteil herstellen und zudem auch toleranter gegenüber Butanol in der Fermentationslösung sind. Die Toleranzgrenze liegt jedoch schon bei 2 Gewichtsprozent Butanol bezogen auf die wässrige Lösung.

Die Extraktion von Alkoholen, insbesondere von Butanol, aus einer wässrigen Lösung, und in einer besonderen Anwendungsform die Aufreinigung von Fermentationsbrühen ist daher weiterhin eine Herausforderung. Destillatives Entfernen der Alkohole aus wässrigen Lösungen ist kostenintensiv. Aufgrund des höheren Siedepunktes von Butanol im Vergleich zu Wasser ist die Rektifikation als Trennverfahren für Butanol nicht wirtschaftlich durchführbar. Bisherig genutzte Extraktionsverfahren benötigen Extraktionsmedien, die in der Regel entflammbar, umweltschädlich oder toxisch sind.

Für die Extraktion von Alkoholen aus Fermentationsbrühen wird zur Zeit am häufigsten der Fettalkohol Oleylalkohol untersucht. Dieser hat jedoch den Nachteil, ein Emulgator zu sein, der bei Verwendung in der Flüssig-Flüssig-Extraktion zu Schäumen führen kann. Eine Schaumphasenbildung führt bei Extraktionskolonnen zu einem höheren Druckverlust und erschwert zudem die Phasentrennung der beiden flüssigen Phasen. Oleylalkohol wird zudem als "reizend für Atemwege" eingestuft.

Es besteht daher ein Bedarf an neuen Extraktionsmedien für die Extraktion von Alkoholen aus wässrigen Lösungen, die alternativ zu den herkömmlichen Verbindungen eingesetzt werden können und die einen hohen Verteilungskoeffizienten und eine hohe Selektivität für den zu extrahierenden Alkohol haben. Die Anforderungen and die neuen Medien sind daher

- gute Selektivität für die Aufnahme des zu extrahierenden Alkohols, idealerweise mit einem Selektivitätskoeffizienten > 100,
- guter Verteilungskoeffizient, beispielsweise mit einem Verteilungskoeffizient $D_{Alkohol} > 2$),
- nicht mischbar mit Wasser,
- geringe Wasseraufnahme von der wässrigen Lösung,
- nicht toxisch für den Mikroorganismus, falls die wässrige Lösung eine Fermentationsbrühe ist.

Aufgabe der vorliegenden Erfindung ist demgemäß die Bereitstellung neuer Extraktionsmedien für die Flüssig-Flüssig-Extraktion von Alkoholen aus wässrigen Lösungen.

Diese Aufgabe wird erfindungsgemäß durch die Merkmale des Hauptanspruchs und der nebengeordneten Ansprüche gelöst.

Überraschend wurde gefunden, dass sich Ionische Flüssigkeiten enthaltend Tricyanomethidanionen in besonderem Maße als Lösungsmittel für eine deartige Flüssig-Flüssig-Extraktion von Alkoholen aus wässrigen Lösungen eignen.

In J.F. Brennecke et al, J. Chem. Eng. Data, 2007, 52, 2462-2467 wird eine Studie beschrieben, in der der

Effekt der Struktur der ionischen Flüssigkeiten, beispielweise die Wahl des Anions, die Alkylkettenlänge am Kation oder das Substitutionsmuster am Kation, auf die Löslichkeit mit Wasser oder mit 1-Octanol untersucht wird.

In A.G. Fadeev, Chem. Commun., 2001, 295-296 wird beschrieben, dass die ionischen Flüssigkeiten 1-Octyl-3-methylimidazoliurn Hexafluorposphat und 1-Butyl-3-methylimidazolium Hexafluorphosphat gute Extraktionsmittel zur Extraktion von Butylalkohol aus Fermentationsbrühen sind.

Gegenstand der Erfindung ist daher ein Verfahren zur Flüssig-Flüssig-Extraktion von Alkoholen aus wässrigen Lösungen unter Verwendung mindestens einer Ionischen Flüssigkeit enthaltend Tricyanomethidanionen als Lösungsmittel nach Anspruch 1.

Idealerweise hat die Ionische Flüssigkeit enthaltend Tricyanomethidanionen die Eigenschaft, mit der wässrigen Lösung enthaltend mindestens einen Alkohol eine zweiphasige Mischung auszubilden.

Die Ionischen Flüssigkeiten mit Tricyanomethidanionen sowie deren Herstellung und Verwendungen als Lösungsmittel für viele synthetische oder katalytische Reaktionen sind bekannt und einige Verbindungen sind kommerziell erhältlich, Lonza Ltd., Schweiz.

Es handelt sich bei dem erfindungsgemäßen Verfahren um eine Flüssig-Flüssig-Extraktion. Bei der Flüssig-Flüssig-Extraktion, bei der zwei Flüssigkeitsphasen beteiligt sind, wird der Wertstoff, in diesem Fall der Alkohol, aus der Trägerphase in die Extraktphase überführt. Zwischen beiden Phasen stellt sich ein Phasengleichgewicht bezüglich der Wertstoffkonzentration nach dem Nemst'schen Verteilungsgesetz ein:

$$D_{Alkohol} = \frac{C^{IL}_{Alkohol}}{C^{AQ}_{Alkohol}}$$

$D_{Alkohol}$ = Nemst'scher Verteilungskoeffizient für den Wertstoff, hier Alkohol $C^{IL}_{Alkohol}$: Konzentration des Wertstoffes, hier Alkohol, in der Ionischen Flüssigkeit

$C^{AQ}_{Alkohol}$: Konzentration des Wertstoffes, hier Alkohol, in der wässrigen Phase

Die Selektivität S wird definiert als Quotient der Nemst'schen Verteilungskoeffizienten von Alkohol zu Wasser:

$$S = \frac{D_{Alkohol}}{D_{Wasser}}$$

Bei der Flüssig-Flüssig-Extraktion handelt es sich um ein Trennverfahren, in dem der Stofftransport zwischen zwei flüssigen Phasen stattfindet und durch das sich einstellende thermodynamische Gleichgewicht laut dem Nernstschen Verteilungskoeffizienten limitiert wird.

Bevorzugt wird die erfindungsgemäße Flüssig-Flüssig-Extraktion von Alkoholen aus wässrigen Lösungen unter Verwendung mindestens einer Ionischen Flüssigkeit enthaltend Tricyanomethidanionen nach Anspruch 1 durchgeführt, indem

a) die wässrige Lösung enthaltend mindestens einen Alkohol bereitgestellt wird,
b) die wässrige Lösung aus a) mit der mindestens einen Ionischen Flüssigkeit enthaltend Tricyanomethidanionen intensiv gemischt wird, so dass die Ionische Flüssigkeit in der Lage ist, mindestens einen Teil des Alkohols aus der wässrigen Lösung zu extrahieren und mit diesem Alkohol eine mindestens einphasige Mischung herzustellen,
c) abtrennen der mindestens einphasigen Mischung aus b) von der wässrigen Lösung,
d) trennen der einphasigen Mischung aus b) in die Komponenten Alkohol und Ionische Flüssigkeit, und gegebenenfalls
e) zurückführen der Ionischen Flüssigkeit aus d) in Schritt b).

Die erfindungsgemäße Flüssig-Flüssig-Extraktion, wie beschrieben, kann im Batch-Verfahren durchgeführt werden. Sie kann aber auch kontinuierlich oder halbkontinuierlich durchgeführt werden. Dabei kann die Flüssig-Flüssig-Extraktion sowohl in der Gegenstrom-, Gleichstrom als auch in der Kreuzstromfahrweise und in einer oder mehrerer Stufen durchgeführt werden. Die Flüssig-Flüssig-Extraktion kann sowohl in einer einstufigen oder mehrstufigen Mixer-settler-Batterie oder aber auch in einer Extraktionkolonne erfolgen. Das erfindungsgemäße Verfahren kann in allen dem Fachmann bekannten Extrakionsapparaturen und Fahrweisen durchgeführt werden, beispielsweise dokumentiert durch die Fachliteratur von J. Rydberg, M. Cox, C. Muskas, G.R. Chopppin, 2004, Solvent Extraction Principles and Practice oder R.H. Perry, 1984, Perry's chemical engineer's handbook.

Eine Variante einer Flüssig-Flüssig-Extraktion ist in Figur 1 beschrieben. Die Bezeichnungen der Figur 1 ist wie folgt:

[1] Reaktionsgefäß enthaltend die wässrige Lösung enthaltend mindestens einen Alkohol und die erfindungsgemäße Ionische Flüssigkeit, insbesondere ein in-situ Fermenter
[2] Rückgewinnungseinheit
[3] Zugabe der wässrigen Lösung enthaltend mindestens einen Alkohol, insbesondere des Fermentationsmediums inklusive Mikroorganismen und Ionische Flüssigkeit
[4] Wässrige Phase der wässrigen Lösung enthaltend mindestens einen Alkohol, insbesondere bei der Fermentation auch die Mikroorganismen
[5] Phase der Ionischen Flüssigkeit, gegebenenfalls enthaltend den Teil des extrahierten Alkohols bildend die mindestens einphasige Mischung
[6] Strom zur Rückgewinnungseinheit enthaltend die Phase der Ionischen Flüssigkeit, enthaltend den Teil des extrahierten Alkohols
[7] Extrahierter und abgetrennter Alkohol
[8] Gegebenenfalls abgeführter Strom der wässrigen Phase, insbesondere der Fermentationsbrühe
[9] Rückführung der aufgereinigten Ionischen Flüssigkeit.

In [1] wird die wässrige Phase enthaltend den mindestens einen Alkohol, insbesondere die Fermentationsbrühe, enthaltend Fermentationsmedium und Mikroorganismen, mit der Ionischen Flüssigkeit in Kontakt gebracht und vermischt. In [1] erfolgt im Falle der bevorzugten Ausführungsform der Fermentation zugleich die Fermentation, d.h. die Produktion des Alkohols durch die Mikroorganismen und Abtrennung des Alkohols aus der Fermentationsbrühe durch Extraktion mittels Ionischer Flüssigkeit. Nach trennen der Phasen in [4] und [5] wird die Ionische Flüssigkeit mittels Strom [6] in eine Rückgewinnungseinheit [2] geführt und das Butanol abgetrennt, welches im Strom [7] abgeführt wird. Im Strom [9] wird die regenerierte Ionische Flüssigkeit in [1] zurückgeführt.

Eine Variante einer erfindungsgemäßen Fermentation mit Hilfe einer Extraktionskolonne ist in Figur 2 beschrieben.
Die Bezeichnung der Figur 2 ist wie folgt:

[1] Fermenter
[2] Rückgewinnungseinheit
[3] Zugabe des Fermentationsmediums inklusive Mikroorganismen
[4] Fermentationsbrühe
[5] Phase der Ionischen Flüssigkeit, gegebenenfalls enthaltend den Teil des extrahierten Alkohols bildend die mindestens einphasige Mischung
[6] Wässrige Phase der wässrigen Lösung enthaltend mindestens einen Alkohol
[7] Abgeführte wässrige Phase, wobei diese in [1] auch zurückgeführt werden kann
[8] Gegebenenfalls abgeführter Strom der wässrigen Phase, Fermentationsbrühe
[9] Extraktionskolonne
[11] Extrahierter und abgetrennter Alkohol
[10] Strom zur Rückgewinnungseinheit enthaltend die Phase der Ionischen Flüssigkeit, enthaltend den Teil des extrahierten Alkohols
[12] Zellrückführung, enthaltend die Mikroorganismen und teilweise Fermentationsmedium
[13] Zellabtrennungseinheit
[14] Wässrige Phase der wässrigen Lösung enthaltend mindestens einen Alkohol
[15] Zugabe der Ionischen Flüssigkeit

In [1] wird die Fermentationsbrühe, enthaltend Fermentationsmedium und Mikroorganismen gerührt und begast. Mittels Strom [4] wird ein Teil der Fermentationsbrühe der Zellabtrennungseinheit zugführt. In der Zellabtrennungseinheit [13] werden die Zellen bzw. Mikroorganismen getrennt. Die Zellen bzw. Mikroorganismen werden mittels Strom [12] in den Fermenter [1] zurückgeführt. Die abgetrennte wässrige Lösung enthaltend mindestens einen Alkohol wird mittels Strom [14] in die Extraktionskolonne [9] geführt. In der Extraktionskolonne [9] werden die beiden Phasen, wässrige Phase enthaltend mindestens einen Alkohol und ionische Flüssigkeit gegebenenfalls enthaltend den Teil des extrahierten Alkohols, in Kontakt gebracht. In der Extraktionskolonne wird der Alkohol extrahiert, d.h. ein teil geht von der wässrigen Phase in die ionische Flüssigkeitsphase über. Die wässrige Phase wird halb-kontinuierlich in Strom [7] abgezogen und teilweise oder aber auch ganz in [1] zurückgeführt. Mittels Strom [10] wird die Phase der Ionischen Flüssigkeit, enthaltend den Teil des extrahierten Alkohols in eine Rückgewinnungseinheit geführt. In der Rückgewinnungseinheit [2] wird der Alkohol von der ionischen Flüssigkeit abgetrennt und in Strom [11] abgeführt. Die regenierierte Strom [8], die regenerierte

Ionische Flüssigkeit enthaltend keinen Alkohol, wird in die Extraktionskolonne [9] zurückgeführt.

Generell umfasst der Begriff Alkohol im Sinne der Erfindung sowohl Monohydroxyalkohole, bevorzugt mit 2, 3 oder 4 C-Atomen, als auch Alkohole mit mehr als einer Hydroxygruppe, beispielsweise Diole, bevorzugt mit 3, 4 oder 5 C-Atomen.

Ausgewählte Diole sind beispielsweise 2,3-Butandiol oder 1,3-Propandiol.

In einer bevorzugten Ausführungsform der Erfindung wird der mindestens eine Alkohol aus der Gruppe Ethanol, Isopropanol, Propanol, n-Butanol oder Isomere des n-Butanol oder Mischungen derselben gewählt. Besonders bevorzugt wird das erfindungsgemäße Verfahren für die Extraktion von n-Butanol, Isomeren des n-Butanol oder Mischungen derselben verwendet.

Das durch Fermentation hergestellte sogenannte Biobutanol enthält als Hauptkomponente n-Butanol und als Nebenbestandteile isomere Butanole. Der Begriff n-Butanol ist gleichbedeutend mit 1-Butanol.

Die wässrige Lösung des erfindungsgemäßen Verfahrens beinhaltet den Alkohol in einer Konzentration von 0,01 bis 50 Gewichtsprozent, bevorzugt in einer Konzentration von 0,1 bis 30 Gewichtsprozent, insbesondere bevorzugt in einer Konzentration von 0,5 bis10 Gewichtsprozent bezogen auf die wässrige Lösung. Für wässrige Lösungen aus Biomasse, d.h. für Fermentationsbrühen, liegt der Alkohol in einer Konzentration von 0,1 bis 3 Gewichtsprozent vor, bevorzugt in einer Konzentration von 0,5 bis 2 Gewichtsprozent vor, bezogen auf die Fermentationsbrühe. Eine natürliche Grenze ist die Produktionsgrenze des Mikroorganismus. Es ist jedoch auch möglich, die Fermentationsbrühe vorher aufzukonzentrieren und dann das erfindungsgemäße Verfahren durchzuführen.

In einer bevorzugten Ausführungsform der Erfindung ist die wässrige Lösung enthaltend mindestens einen Alkohol eine Fermentationsbrühe, insbesondere eine Fermentationsbrühe aus einer Aceton-Butanol-Ethanol-Fermentation (ABE-Fermentation).

Für die ABE-Fermentation wurde der anfangs verwendete Mikroorganismus *Clostridium beijerinckii* weiterentwickelt zum *Clostridium beijerinckii BA109,* der eine Butanolkonzentration von bis zu 17,8 g/l produzieren bzw. tolerieren kann und zwar mit im Vergleich zu definierten Nährmedien, beispielsweise günstigerem Glukose-Corn Steep Liquor-Medium. Die Konzentration des Glukose-Corn Steep Liquor-Medium beträgt 60g/l. Wichtige Nebenprodukte bei dieser Fermentation sind Aceton und Ethanol mit Konzentrationen von 5,5 g/l und 1 g/l. Weitere Nebenprodukte mit sehr geringen Konzentrationen sind Essigsäure und Buttersäure.

Es gehört zum generellen Fachwissen, bei welcher Temperatur die erfindungsgemäße Flüssig-Flüssig-Extraktion durchgeführt wird. Im Falle der besonderen Ausführungsform der Extraktion des Alkohols aus einer in-situ-Fermentation, wie später definiert, ist beispielsweise die ideale Temperatur des Mikroorganismus zu beachten, bei der die Produktion des Alkohols bevorzugt stattfinden kann.

In einer bevorzugten Ausführungsform der vorliegenden Erfindung ist das Kation der Ionischen Flüssigkeit enthaltend Tricyanomethidanionen hydrophob.

Die ionische Flüssigkeit enthaltend Tricyanomethidanionen ist ausgewählt aus der Gruppe der Verbindungen der Formeln (1), (2) oder (3)

$$[NR_4]^+ [C(CN)_3]^- \quad (1),$$

$$[PR_4]^+ [C(CN)_3]^- \quad (2),$$

$$[SR_3]^+ [C(CN)_3]^- \quad (3),$$

wobei

R jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet, mit der Bedingung, dass mindestens zwei Substituenten R mindestens 5 C-Atome haben, oder ausgewählt

aus der Gruppe der Verbindungen der Formeln (4) oder (5),

$$[C(NR^3R^4)(OR^5)(NR^6R^7)]^+ [C(CN)_3]^- \quad (4),$$

$$[C(NR^3R^4)(SR^5)(NR^6R^7)]^+ [C(CN)_3]^- \quad (5),$$

wobei
$R^3$ bis $R^7$ jeweils unabhängig voneinander

- H, wobei H für $R^5$ ausgeschlossen wird,
- ein geradkettiges oder verzweigtes Alkyl mit 1 bis 20 C-Atomen, geradkettiges oder verzweigtes Alkenyl mit 2-20 C-Atomen und einer oder mehreren Doppelbindungen,
- geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet, oder ausgewählt

aus der Gruppe der Verbindungen der Formel (6),

$$[C(NR^8R^9)(NR^{10}R^{11})(NR^{12}R^{13})]^+ [C(CN)_3]^- \qquad (6),$$

wobei
$R^8$ bis $R^{13}$ jeweils unabhängig voneinander

- H,
- ein geradkettiges oder verzweigtes Alkyl mit 1 bis 20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet, oder ausgewählt

aus der Gruppe der Verbindungen der Formel (7),

$$[HetN]^{z+} [C(CN)_3]^- \qquad (7),$$

wobei

- $HetN^{z+}$ ein heterocyclisches Kation, ausgewählt aus der Gruppe

Imidazolium

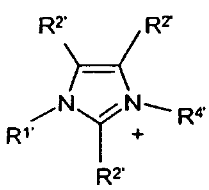

,

1H-Pyrazolium

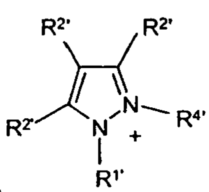

,

3H-Pyrazolium

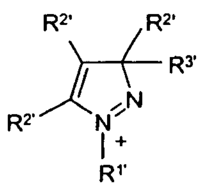

,

4H-Pyrazolium

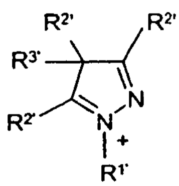

,

1-Pyrazolinium

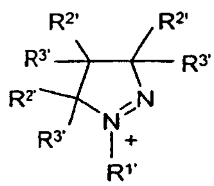

,

2-Pyrazolinium

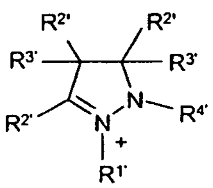

,

3-Pyrazolinium

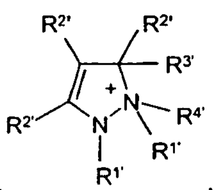

,

2,3-Dihydro-Imidazolinium

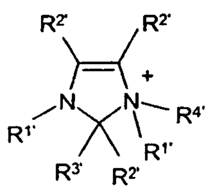

,

4,5-Dihydro-Imidazolinium

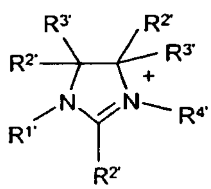

,

2,5-Dihydro-Imidazolinium

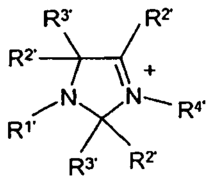

,

Pyrrolidinium

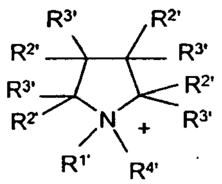

,

1,2,4-Triazolium

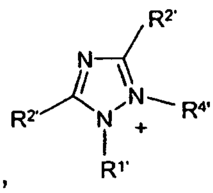

,

1,2,4-Triazolium

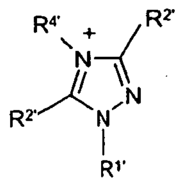

,

1,2,3-Triazolium

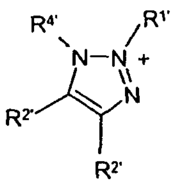

,

1,2,3-Triazolium

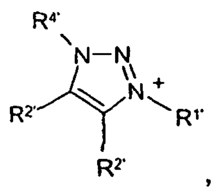

,

Pyridinium

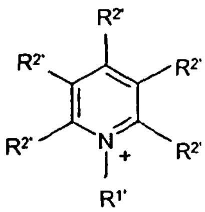

,

Pyridazinium

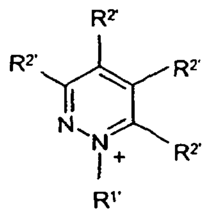

,

Pyrimidinium

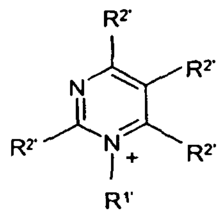

,

Piperidinium

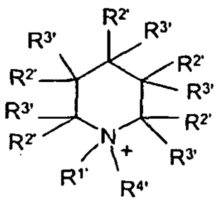

,

Morpholinium

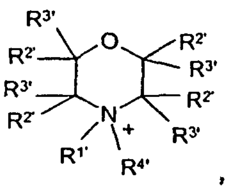

,

Piperazinium

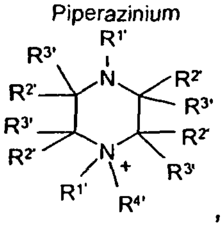

,

Piperazinium

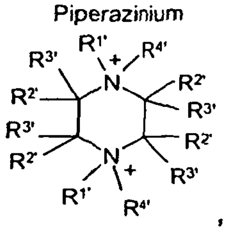

,

Pyrazinium

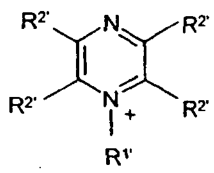

,

Thiazolium

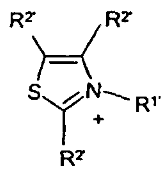

,

Oxazolium

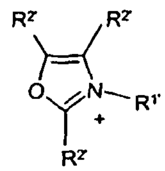

,

Indolium

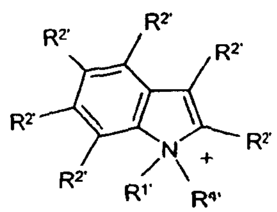

,,

Chinolinium

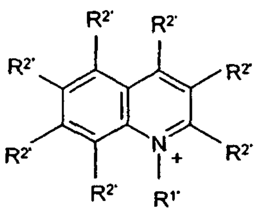

,

Isochinolinium

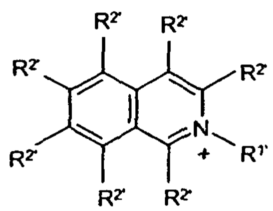

,

Chinoxalinium

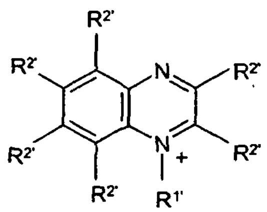

oder

Indolinium

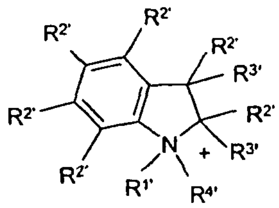

bedeutet, wobei die Substituenten

$R^{1'}$ bis $R^{4'}$ jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann,

bedeutet,
wobei die Substituenten $R^{1'}$, $R^{2'}$, $R^{3'}$ und/oder $R^{4'}$ zusammen auch ein Ringsystem bilden können.

Unter vollständig ungesättigten Substituenten werden im Sinne der vorliegenden Erfindung auch aromatische Substituenten verstanden.

Als Substituenten R und $R^3$ bis $R^{13}$ der Verbindungen der Formeln (1) bis (6) kommen erfindungsgemäß jeweils unabhänig voneinander bevorzugt in Frage $C_6$- bis $C_{18}$-, insbesondere $C_8$- bis $C_{14}$-Alkylgruppen.

Die Substituenten R in den Verbindungen der Formel (1), (2) oder (3) können dabei gleich oder verschieden sein. Für Ammonium- oder Phosphonium-Tricyanomethide der Formeln (1) oder (2) sind bevorzugt drei Substituenten R gleich und ein Substituent R ist verschieden. Für die Sulfonium-Tricyanomethide der Formel (3) sind zwei Substituenten R bevorzugt gleich und ein Substituent R ist verschieden.

Die Substituenten R sind insbesondere bevorzugt Pentyl, Hexyl, Octyl, Decyl, Dodecyl oder Tetradecyl.

Bis zu vier Substituenten des Guanidinium-Kations $[C(NR^8R^9)(NR^{10}R^{11})(NR^{12}R^{13})]^+$ können auch paarweise derart verbunden sein, dass mono-, bi- oder polycyclische Kationen entstehen.
Ohne Einschränkung der Allgemeinheit sind Beispiele für solche Guanidinium-Kationen:

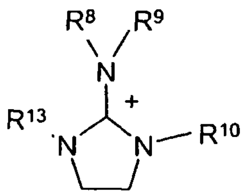 , 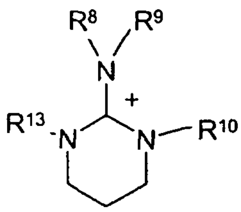 , 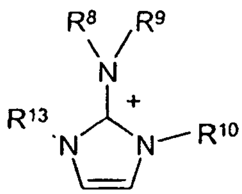 , 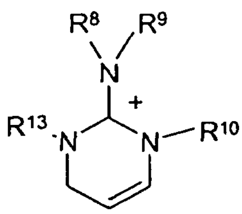 ,

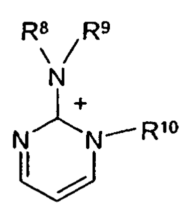 , 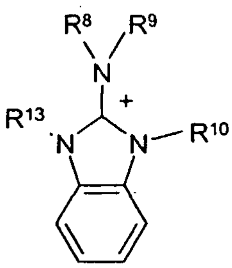 , 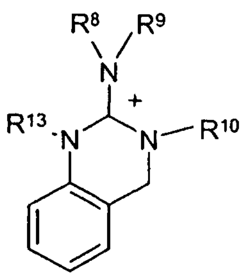 , 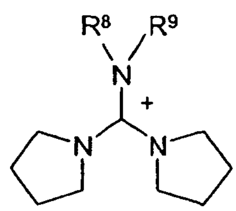

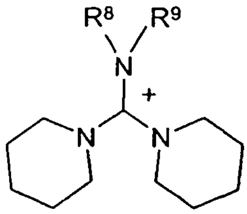 oder 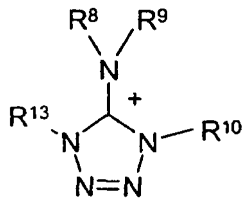

wobei die Substituenten $R^8$ bis $R^{10}$ und $R^{13}$ eine zuvor angegebene oder besonders bevorzugte Bedeutung haben können.

Gegebenenfalls können die Carbocyclen oder Heterocyclen der zuvor angegebenen Guanidinium-Kationen noch durch $C_1$- bis $C_6$-Alkyl substituiert sein.

Bis zu vier Substituenten des Uroniumkations $[C(NR^3R^4)(OR^5)(NR^6R^7)]^+$ oder des Thiouroniumkations $[C(NR^3R^4)(SR^5)(NR^6R^7)]^+$ können auch paarweise derart verbunden sein, dass mono-, bi- oder polycyclische Kationen entstehen.

Ohne Einschränkung der Allgemeinheit sind Beispiele für solche Kationen im Folgenden angegeben, wobei Y = O oder S bedeutet:

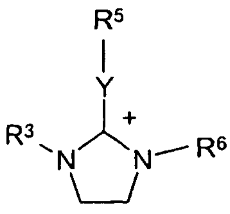 , 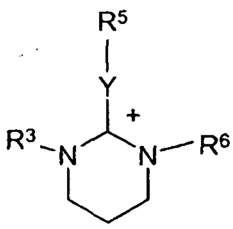 , 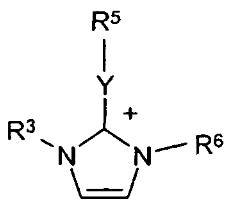 , 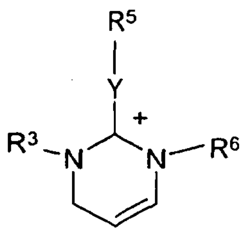 ,

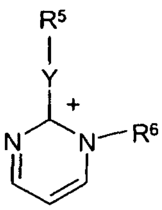 , 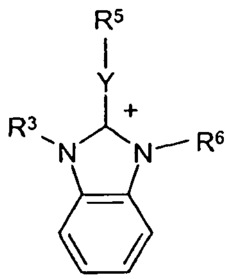 , 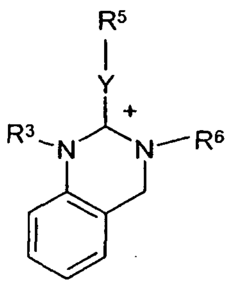 , 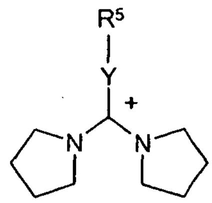

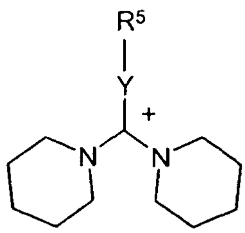 oder 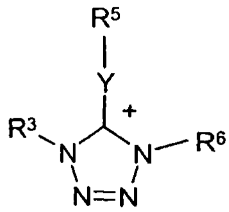 ,

wobei die Substituenten $R^3$, $R^5$ und $R^6$ eine zuvor angegebene oder besonders bevorzugte Bedeutung haben können. Gegebenenfalls können die Carbocyclen oder Heterocyclen der zuvor angegebenen Kationen noch durch $C_1$- bis $C_6$-Alkyl substituiert sein.

Die Substituenten $R^3$ bis $R^{13}$ sind jeweils unabhängig voneinander bevorzugt eine geradkettige oder verzweigte Alkylgruppe mit 1 bis 16 C-Atomen. Die Substituenten $R^3$ und $R^4$, $R^6$ und $R^7$, $R^8$ und $R^9$, $R^{10}$ und $R^{11}$ und $R^{12}$ und $R^{13}$ in Verbindungen der Formeln (4) bis (6) können dabei gleich oder verschieden sein. Besonders bevorzugt sind $R^3$ bis $R^{13}$ jeweils unabhängig voneinander Methyl, Ethyl, n-Propyl, Isopropyl, n-Butyl, tert.-Butyl, sek.-Butyl, Phenyl, Hexyl oder Cyclohexyl, ganz besonders bevorzugt Methyl, Ethyl, n-Propyl, Isopropyl, n-Butyl oder Hexyl.

Als Substituenten $R^{1'}$ bis $R^{4'}$ von Verbindungen der Formel (7) kommen erfindungsgemäß dabei neben H bevorzugt in Frage: $C_1$- bis $C_{20}$, insbesondere $C_1$- bis $C_{12}$-Alkylgruppen.

Die Substituenten $R^{1'}$ und $R^{4'}$ sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Dodecyl, Cyclohexyl, Phenyl oder Benzyl. Sie sind ganz besonders bevorzugt Methyl, Octyl, Decyl oder Dodecyl. In Pyrrolidinium-, Piperidinium- oder Morpholinium-Verbindungen sind die beiden Substituenten $R^{1'}$ und $R^{4'}$ bevorzugt unterschiedlich.

Der Substituent $R^{2'}$ oder $R^{3'}$ ist jeweils unabhängig voneinander insbesondere H, Methyl, Ethyl, Isopropyl, Propyl, Butyl, sek.-Butyl, tert.-Butyl, Cyclohexyl, Phenyl oder Benzyl. Besonders bevorzugt ist $R^{2'}$ H, Methyl, Ethyl, Isopropyl, Propyl, Butyl oder sek.-Butyl. Ganz besonders bevorzugt sind $R^{2'}$ und $R^{3'}$ H.

Die $C_1$-$C_{12}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, $-C_9H_{17}$, $-C_{10}H_{19}$ bis $-C_{20}H_{39}$; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, $-C_9H_{15}$, $-C_{10}H_{17}$ bis $-C_{20}H_{37}$, vorzugsweise Ethinyl, 1- oder 2-Propinyl, 2- oder 3-Butinyl, 4-Pentinyl, 3-Pentinyl oder Hexinyl.

Unsubstituierte gesättigte oder teilweise oder vollständig ungesättigte Cycloalkylgruppen mit 5-7 C-Atomen sind daher Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit $C_1$- bis $C_6$-Alkylgruppen substituiert sein können.

$HetN^{z+}$ ist bevorzugt

Imidazolium

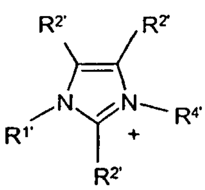

,

Pyrazolium

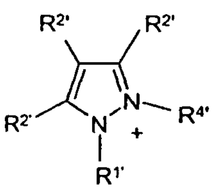

,

Pyrrolidinium

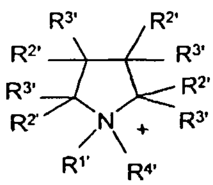

,

Pyridinium

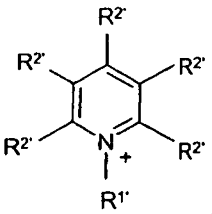

,

Pyrimidinium

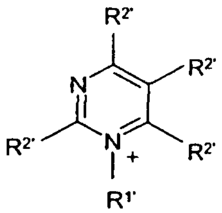

,

Piperidinium

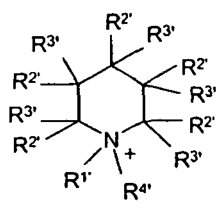

oder

Indolinium

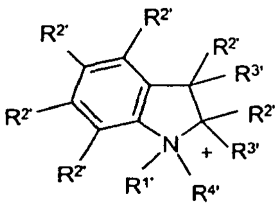

,

wobei die Substituenten $R^{1'}$ bis $R^{4'}$ jeweils unabhängig voneinander eine zuvor beschriebene Bedeutung haben.

Ganz besonders bevorzugt ist $HetN^{z+}$

Imidazolium

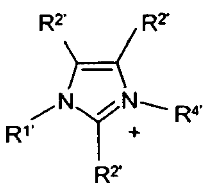

,

Pyrrolidinium

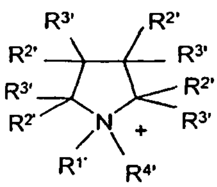

,

Pyridinium

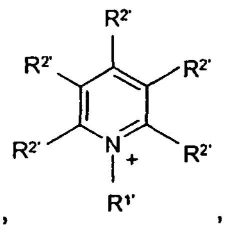

,

Piperidinium

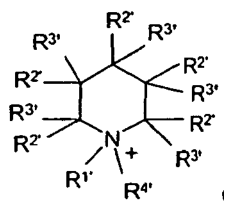

,

oder,

Morpholinium

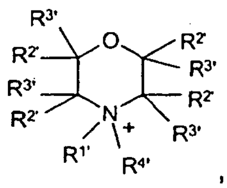

,

wobei die Substituenten $R^{1'}$ bis $R^{4'}$ jeweils unabhängig voneinander eine zuvor beschriebene Bedeutung haben.

$HetN^{z+}$ ist ganz besonders bevorzugt Imidazolium

Imidazolium

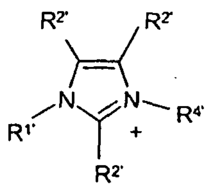

,

wobei die Substituenten $R^{1'}$ bis $R^{4'}$ jeweils unabhängig voneinander eine zuvor beschriebene Bedeutung haben.

In einer bevorzugten Ausführungsform des Verfahrens wird die mindestens eine Ionische Flüssigkeit enthaltend Tricyanomethidanionen ausgewählt aus der Gruppe der Verbindungen der Formeln (1), (2) oder (7), mit Substituenten, wie zuvor definiert oder bevorzugt definiert. Ganz besonders bevorzugt werden Verbindungen der Formeln (1) oder (2) und deren bevorzugte Verbindungen eingesetzt, wie zuvor beschrieben.

Besonders bevorzugt werden die Ionischen Flüssigkeiten enthaltend Tricyanomethidanionen für den Einsatz im erfindungsgemäßen Verfahren ausgewählt aus der Gruppe
1-Octyl-3-methylimidazolium Tricyanomethid,
1-Decyl-3-methylimidazolium Tricyanomethid,
1-Dodecyl-3-methylimidazolium Tricyanomethid,
Trihexyl-tetradecyl-ammonium Tricyanomethid,
Trihexyl-tetradecyl-phosphonium Tricyanomethid,
N-Octylpyridinium Tricyanomethid,
1-Octyl-1-methyl-pyrrolidinium Tricyanomethid,
N-Octyl-N-methyl-morpholinium Tricyanomethid,
1-Octyl-1-methyl-piperidinium Tricyanomethid.

Ganz besonders bevorzugt hinsichtlich der Selektivität sind die ionischen Flüssigkeiten gemäß der Formeln (1) oder (2), wie zuvor definiert oder als bevorzugt beschrieben, besonders bevorzugt Trihexyl-tetradecyl-phosphonium Tricyanomethid oder Trihexyl-tetradecyl-ammonium Tricyanomethid. Die Selektivitäten zu Butanol sind, insbesondere für die genannten Einzelverbindungen, vergleichbar mit der Selektivität von Oleylalkohol. Weitere allgemeine Aussagen diesbezüglich sind auch im Beispielteil enthalten.

Es versteht sich für den Fachmann von selbst, dass in den erfindungsgemäßen Verbindungen Substituenten wie beispielsweise C, H, N, O, Cl, F durch die entsprechenden Isotope ersetzt sein können.

Die Bereitstellung der wässrigen Lösung enthaltend mindestens einen Alkohol des erfindungsgemäßen Verfahrens gehört zum allgemeinen Fachwissen. Es kann die wässrige Lösung sowohl gezielt hergestellt werden, als auch eine wässrige Lösung aus einem Produktionsprozess eingesetzt werden. Im besonderen Falle der Fermentationsbrühe ist der Produktionsprozess eine Fermentation.

Eine intensive Mischung kann vorzugsweise unter Rühren stattfinden. Es sind jedoch auch alle anderen Arten von Vermischungen möglich, beispielsweise physikalische Vorgänge wie Schütteln oder Ultraschall.

Das Abtrennen der mindestens einphasigen Mischung aus Schritt b) des erfindungsgemäßen Verfahrens von der wässrigen Lösung erfolgt nach Methoden, die dem Fachmann bekannt sind.
Wird das erfindungsgemäße Verfahren im Batch-Betrieb genutzt, so entsteht nach Beendigung des Rührens eine Entmischung der wässrigen Lösung von der mindestens einphasigen Mischung enthaltend mindestens einen Teil des zu extrahierenden Alkohols, wie zuvor definiert und die mindestens eine Ionische Flüssigkeit und die untere Phase kann

beispielsweise durch Entnahme am Boden des Reaktionsgefäßes abgetrennt werden.

Bei kontinuierlicher Nutzung des Verfahrens wird ebenfalls am Boden des Reaktionsgefäßes kontinuierlich ein Teil der unteren Phase abgezogen. Verwiesen wird insbesondere auf das Fachwissen, dokumentiert durch beispielsweise die Fachliteratur von J. Rydberg, M. Cox, C. Muskas, G.R. Chopppin, 2004, Solvent Extraction Principles and Practice oder R.H. Perry, 1984, Perry's chemical engineer's handbook.

Die obere Phase in diesem Abtrennprozess ist in der Regel die mindestens mehrphasige Mischung der mindestens einen Ionischen Flüssigkeit mit dem extrahierten Teil des Alkohols, wie zuvor beschrieben.

Die Abtrennung der Komponente Alkohol von der Ionischen Flüssigkeit erfolgt nach Methoden, die dem Fachmann bekannt sind, beispielweise durch Destillation des Alkohols, Strippen, Flash, Verdampfung, Adsorption oder chromatographischen Methoden.

Die aufbereitete Ionische Flüssigkeit, wie zuvor beschrieben, kann gegebenenfalls in das erfindungsgemäße Verfahren zurückgeführt werden und steht wieder als Lösungsmittel zur Verfügung.

Das erfindungsgemäße Verfahren zur Flüssig-Flüssig-Extraktion kann im kontinuierlichen aber auch im halbkontinuierlichen Betrieb durchgeführt werden, wie zuvor beschrieben. Die Aufreinigung der Flüssig-Flüssig-Extraktion kann dabei sowohl in-situ als auch entkoppelt stattfinden. Bei der in-situ-Extraktion wird gleichzeitig fermentiert und der Wertstoff, hier erfindungsgemäß mindestens ein Alkohol, wie zuvor beschrieben, abgetrennt, in dem die Fermentationsbrühe direkt mit der Ionischen Flüssigkeit in Kontakt gebracht wird. Somit wird der Wertstoff aus der wässrigen Phase entfernt und die Konzentration des Wertstoffes niedrig gehalten, so dass dieser die Mikroorganismen nicht inhibiert. Inhibierung bedeutet, dass der Wachstum der Mikroorganismen verlangsamt oder gar gestoppt wird, wodurch auch die Produktion des Wertstoffes verlangsamt oder gar gestoppt wird.

Das erfindungsgemäße Verfahren, wie zuvor beschrieben, kann in jeder geeigneten Apparatur durchgeführt werden, wie Sie dem Fachmann bekannt sind.

Ebenfalls Gegenstand der Erfindung ist die Verwendung mindestens einer Ionischen Flüssigkeit enthaltend Tricyanomethidanionen als Lösungsmittel für eine Flüssig-Flüssig-Extraktion von Alkoholen aus wässrigen Lösungen.

Es gelten für diesen Gegenstand der Erfindung ebenfalls alle Ausführungen hinsichtlich der bevorzugten Ausführungsformen des Verfahrens der Flüssig-Flüssig-Extraktion, der wässrigen Lösung, des Alkohols sowie der mindestens einen Ionischen Flüssigkeit.

Bevorzugte Merkmalskombinationen der Erfindung sind in den Ansprüchen offenbart.

Auch ohne weitere Ausführungen wird davon ausgegangen, dass ein Fachmann die obige Beschreibung im weitesten Umfang nutzen kann.

**Beispiele:**

Die Synthese der Ionischen Flüssigkeiten enthaltend Tricyanomethidanionen kann beispielsweise nach der Offenbarung der WO 2006/021390 erfolgen oder die Ionischen Flüssigkeiten sind kommerziell erhältlich.

Beispiele zur Synthese ausgewählter Verbindungen sind:

Beispiel A: Synthese von Trihexyl-tetradecyl-ammonium Tricyanomethid

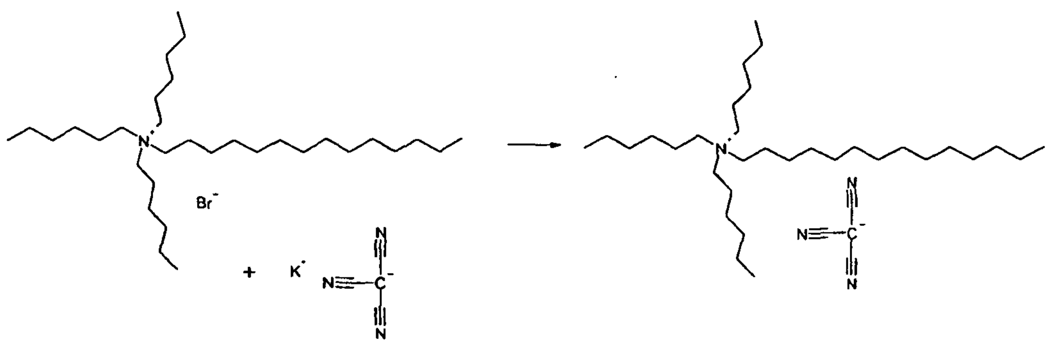

Es werden 100 g Trihexyl-tetradecyl-ammonium bromid in 400 L VE-Wasser gelöst und anschließend langsam mit 26 g KaliumTricyanomethid versetzt. Dieses Reaktionsgemisch wird noch für weitere 2 Stunden (h) bei Raumtemperatur nachgerührt und über Nacht stehen gelassen.

Die Aufarbeitung erfolgt durch Extraktion mit Dichlormethan. Die organische Phase wird dann mit VE-Wasser

Bromid-frei gewaschen. Die organische Lösung wird mit 8 g ALOX und 5 g Aktivkohle versetzt, filtriert und anschließend am Rotationsverdampfer bei einem Wasserbad von ca. 80 °C eingeengt.
1 H NMR (d6-DMSO): δ = 3.1 (m, 6H), 2.51 (m, 2H), 1.58 (m, 8H), 1.30 (m, 24H), 1.26 (m, 16H), 0.89 (m, 12H).

Beispiel B: Synthese von Trihexyl-tetradecyl-phosphonium Tricyanomethid

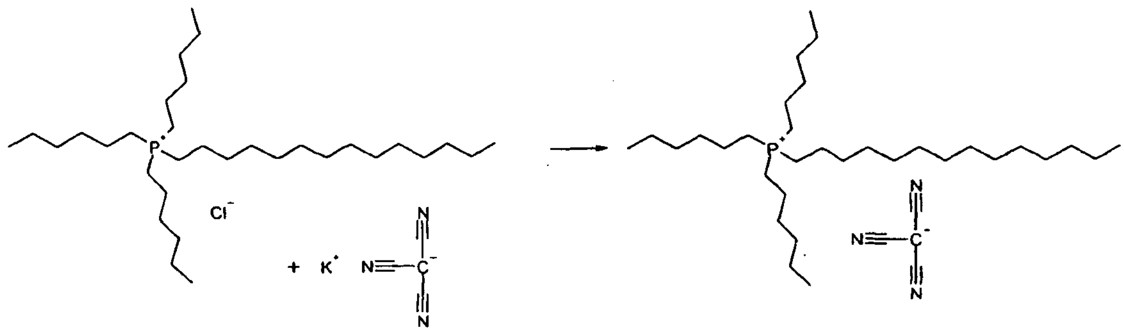

Analog zu Beispiel A werden 100 g Trihexyl-tetradecyl-phosphonium chlorid mit 28 g KaliumTricyanomethid versetzt und entsprechend aufgearbeitet.
1 H NMR (d6-DMSO): δ = 2.51 (m, 2H), 2.18 (m, 6H), 1.47 (m, 8H), 1.39 (m, 8H), 1.30 (m, 14H), 1.26 (m, 18H), 0.89 (m, 12H).

Beispiel C: Synthese von 1-Methyl-1-octylpyrrolidinium Tricyanomethid

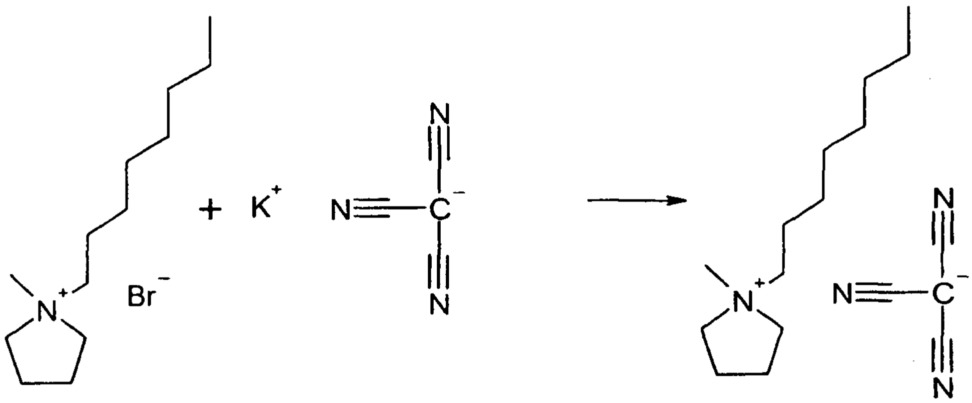

Analog zu Beispiel A werden 100 g 1-Methyl-1-octyl-pyrrolidinium bromid mit 51 g KaliumTricyanomethid versetzt und entsprechend aufgearbeitet.
1 H NMR (d6-DMSO): ö = 3.20 (m, 4H), 3.05 (m, 4H), 2.74 (s, 3H), 1.84 (m, 2H), 1.45 (m, 2H), 1.05 (m, 10H), 0.63 (t, J(H,H) = 6.6 Hz, 3H).

Beispiel D: Synthese von N-Methyl-N-octylmorpholinium Tricyanomethid

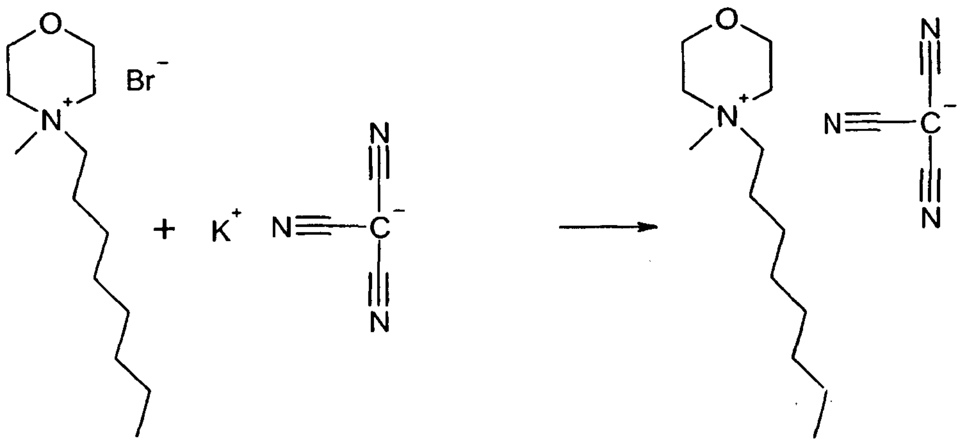

Analog zu Beispiel A werden 100 g 4-Methyl-4-octyl-morpholinium bromid mit 49 g KaliumTricyanomethid versetzt und entsprechend aufgearbeitet.
1 H NMR (d6-DMSO): δ = 3.67 (m, 4H), 3.15 (m, 4H), 2.88 (s, 3H), 2.71 (t, J(H,H) = 2.0 Hz, 2H), 1.44 (m, 2H), 1.06 (m, 10H), 0.64 (t, J(H,H) = 7.0 Hz, 3H).

Beispiel E: Synthese von 1-Methyl-1-octylpiperidinium Tricyanomethid

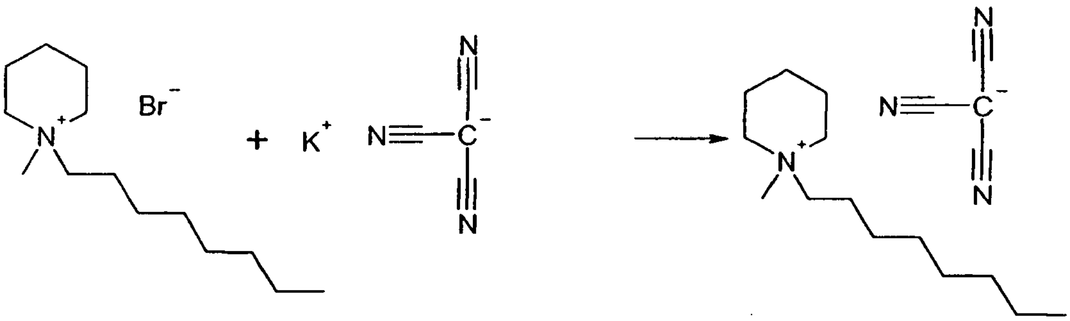

Analog zu Beispiel A werden 100 g 1-Methyl-1-octyl-piperidinium bromid mit 49 g KaliumTricyanomethid versetzt und entsprechend aufgearbeitet.
1 H NMR (d6-DMSO): δ = 3.33 (m, 4H), 3.28 (m, 6H), 2.99 (s, 3H), 2.50 (t, J(H,H) = 2.0 Hz, 2H), 2.12 (m, 2H), 1.30 (m, 10H), 0.88 (t, J(H,H) = 7.0 Hz, 3H).

Beispiel F: Synthese von 1-Octyl-3-methyl-imidazolium Tricyanomethid

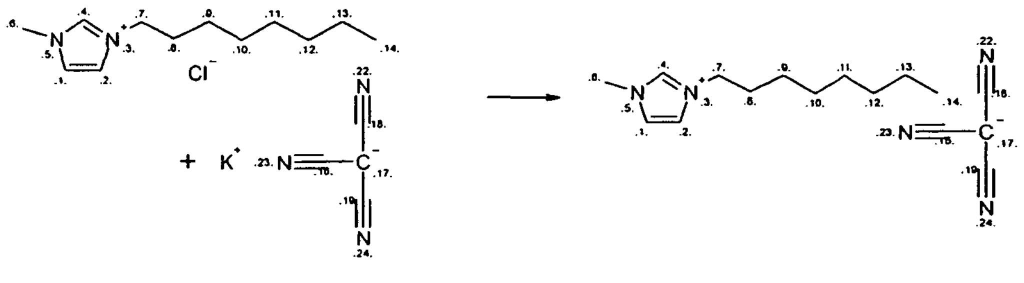

Analog zu Beispiel A werden 250 g 3-Methyl-1-octyl-imidazolium chlorid mit 153 g KaliumTricyanomethid versetzt und entsprechend aufgearbeitet.

1H NMR (d6-DMSO): δ = 9.10 (s, 1H), 7.76 (t, J(H,H) = 1.4 Hz, 1 H), 7.70 (t, J(H,H) = 1.4 Hz, 1 H), 4.15 (t, J(H,H) = 7.4 Hz, 2H), 3.85 (s, 3H), 1.25 (m, 12H), 0.86 (t, J(H,H) = 6.6 Hz, 3H).

Beispiel G: Synthese von N-Octylpyridinium Tricyanomethid

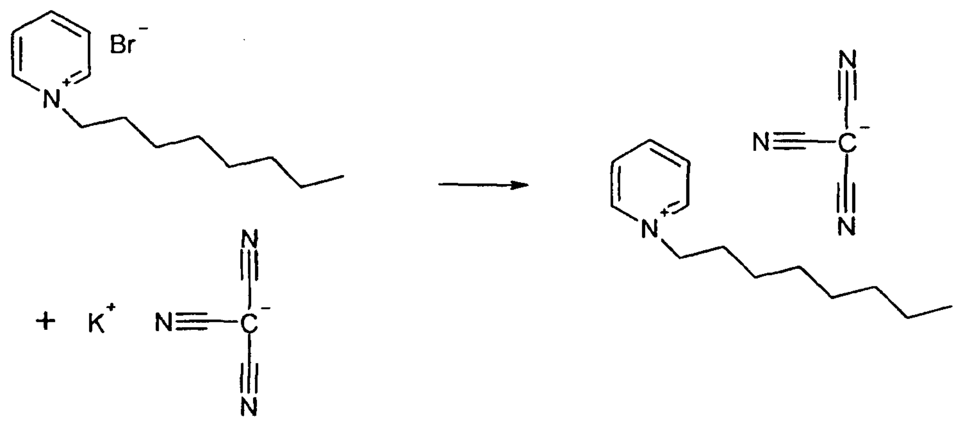

Analog zu Beispiel A werden 134 g 1-Octylpyridinium bromid mit 70 g KaliumTricyanomethid versetzt und entsprechend aufgearbeitet.
1 H NMR (d6-DMSO): δ = 9.10 (d, J(H,H) = 5.2 Hz, 2H), 8.61 (t, J(H,H) = 8.7 Hz, 1 H), 8.16 (t, J(H,H) = 6.6 Hz, 2H), 4.60 (t, J(H,H) = 8.5 Hz, 2H), 1.92 (m, 2H), 1.27 (m, 10H), 0.86 (t, J(H,H) = 7.0 Hz, 3H).

**Beispiel 1:**

**Durchführung - Variante 1:**

Die Messung des Verteilungskoeffizienten erfolgt in doppelwandigen Glassgefäßen mit einem maximalen Volumen von 10 ml. Die Anfangskonzentration von Butanol in der wässrige Phase ist 1 Gew.%. Gleiche Massen (3 g) von jeder Phase werden in Kontakt gebracht und mittels eines Magnetrührers (Variomag telesystem 06.07) bei konstanter Temperatur (25°C) 24 h intensiv vermischt. Durch die lange Versuchsdauer wird das Erreichen des Gleichgewichts gewährleistet. Die Temperierung erfolgt mittels eines Kryostaten (Julabo F25 ME). Nach 10 Minuten Phasentrennung werden Proben von jeder Phase entnommen und analysiert.
Die zur Zeit am meisten untersuchte Substanz Oleylalkohol erreicht in dieser Untersuchung nach Variante 1 einen Verteifungskoeffizienten von 3,4 und eine Selektivität von 208.

**Durchführung - Variante 2:**

Es werden 0,9 g Ionische Flüssigkeit in einem 2ml Eppendorf Kunststoff-Gefäß eingewogen. Dazu werden 0,9 g 1 % ige wässrige Butanol-Lösung gegeben. Die Lösung wird vorher direkt hergestellt. Die Probentubes werden bei 25°C im Eppendorf Thermomixer Komfort für 24 h mit maximaler Geschwindigkeit geschüttelt. Danach werden sie für 24 h bei 25°C stehen gelassen, um eine gute Phasentrennung zu erreichen. Die wässrige Phase und die Phase enthaltend die jeweils untersuchte ionische Flüssigkeit werden separiert und jeweils noch einmal in der Eppendorf Laborzentrifuge für 2 min bei 14500 rpm zentrifugiert. Der Gehalt an Butanol in den beiden Phasen wird mittels GC-Headspace aus DMSO-Matrix bestimmt. Der Gehalt an Wasser in der Phase enthaltend die ionische Flüssigkeit wird mittels Karl-Fischer-Titration bestimmt. Der Gehalt an Ionischer Flüssigkeit in der wässrigen Phase wird mittlels HPLC bestimmt.
Untersucht werden nach Variante 2 die folgenden ionischen Flüssigkeiten:

[1] OMIM FAP (1-Octyl-3-methyl-imidazolium Tris(pentafluorethyl)trifluorphosphat),
[2] OMIM PF6 (1-Octyl-3-methyl-imidazolium Hexäfluorphoshat),
[3] OMIM NTF (1-Octyl-3-methyl-imidazolium Bis(trifluormethylsulfonyl)imid,
[4] OMIM BF4 (1-Octyl-3-methyl-imidazolium Tetrafluorborat),
[5] OMIM TCB (1-Octyl-3-methyl-imidazolium Tetracyanoborat),
[6] OMIM TCM (1-Octyl-3-methyl-imidazolium Tricyanomethid),

[7] OMPL TCM (1-Methyl-1-octyl-pyrrolidinium Tricyanomethid),
[8] OMPI TCM (1-Methyl-1octyl-piperidinium Tricyanomethid),
[9] OMMO TCM (N-Octyl-N-methyl-morpholinium Tricyanomethid)
[10] PH3T TCM (Trihexyl-tetradecyl-phosphonium Tricyanomethid)
[11] NH3T TCM (Trihexyl-tetradecyl-ammonium Tricyanomethid).

Die folgende Tabelle fasst die Ergebnisse zusammen: Weiterhin wird jeweils der Mittelwert aus zwei oder drei Werten gelistet:

| Ionische Flüssigkeit | $D_{Bu/IL}$ ($[Bu]_{IL}/[Bu]_{H2O}$) | $S(D_{Bu/IL}/D_{Bu/H2O})$ |
|---|---|---|
| OMIM FAP | 0,52 | 266 |
| OMIM PF6 | 0,92 | 55 |
| OMIM NTF | 1,51 | 135 |
| OMIM BF4 | 2,65 | 19 |
| OMIM TCB | 2,95 | 73 |
| OMIM TCM | 4,74 | 35 |
| OMPL TCM | 4,51 | 38 |
| OMPI TCM | 4,62 | 45 |
| OMMO TCM | 4,09 | 28 |
| PH3T TCM | 3,38 | 240 |
| NH3T TCM | 4,07 | 219 |
| Oleylalkohol | 3,5 | 230 |

• IL = Abkürzung für Ionische Flüssigkeit, Bu = Abkürzung für Butanol

Figur 3 fasst die Ergebnisse für die Tricyanomethide graphisch zusammen. Figur 4 fasst die Ergebnisse für alle Messwerte zusammen.

Die Ergebnisse zeigen, dass ionische Flüssigkeiten mit TCM-Anionen hervorragend für die Extraktion von Butanol aus einer wässrigen Lösung geeignet sind.
Der Vergleich zeigt, dass die ionischen Flüssigkeiten mit Tricyanomethidanionen Alternativen zur Verwendung von ionischen Flüssigkeiten mit Tetracyanoboratanionen sind, gemessen an OMIM TCB. OMIM TCM zeigt den größten Wert bezüglich des Verteilungskoeffizienten im Vergleich zu den genannten ionischen Flüssigkeiten.
Ammonium- oder Phosphonium Tricyanomethide, wie im allgemeinen Teil beschrieben, sind insbesondere geeignet, da sie sowohl eine hohe Selektivität als auch einen hohen Verteilungskoeffizienten besitzen.

Figurenverzeichnis:

Figur 1: Fließbild einer Flüssig-Flüssig-Extraktion
Figur 2: Fließbild einer Extraktionskolonne zur Flüssig-Flüssig-Extraktion
Figur 3: Graphik der Selektivität gegen Verteilungskoeffizienten für Ionische Flüssigkeiten mit Tricyanomethidanion für Butanol
Figur 4: Graphik der Selektivität gegen Verteilungskoeffizienten für die Ionischen Flüssigkeiten [1] bis [11] für Butanol

## Patentansprüche

**1.** Verfahren zur Flüssig-Flüssig-Extraktion von Alkoholen aus wässrigen Lösungen, wobei

a) die wässrige Lösung enthaltend mindestens einen Alkohol bereitgestellt wird,
b) die wässrige Lösung aus a) mit der mindestens einen Ionischen Flüssigkeit enthaltend Tricyanomethidanionen intensiv gemischt wird, so dass die Ionische Flüssigkeit in der Lage ist, mindestens einen Teil des Alkohols aus der wässrigen Lösung zu extrahieren und mit diesem Alkohol eine mindestens einphasige Mischung herzustellen,
c) abtrennen der mindestens einphasigen Mischung aus b) von der wässrigen Lösung,
d) trennen der einphasigen Mischung aus b) in die Komponenten Alkohol und Ionische Flüssigkeit, und gegebenenfalls
e) zurückführen der Ionischen Flüssigkeit aus d) in Schritt b) und wobei die mindestens eine Ionische Flüssigkeit enthaltend Tricyanomethidanionen ausgewählt wird aus der Gruppe der Verbindungen der Formeln (1), (2)

oder (3),

$[NR_4]^+ [C(CN)_3]^-$ (1),

$[PR_4]^+ [C(CN)_3]^-$ (2),

$[SR_3]^+ [C(CN)_3]^-$ (3),

wobei
R jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet, mit der Bedingung, dass mindestens zwei Substituenten R mindestens 5 C-Atome haben

oder ausgewählt wird aus der Gruppe der Verbindungen der Formeln der Formeln (4) oder (5),

$[C(NR^3R^4)(OR^5)(NR^6R^7)]^+ [C(CN)_3]^-$ (4),

$[C(NR^3R^4)(SR^5)(NR^6R^7)]^+ [C(CN)_3]^-$ (5),

wobei
$R^3$ bis $R^7$ jeweils unabhängig voneinander

- H, wobei H für $R^5$ ausgeschlossen wird,
- ein geradkettiges oder verzweigtes Alkyl mit 1 bis 20 C-Atomen, geradkettiges oder verzweigtes Alkenyl mit 2-20 C-Atomen und einer oder mehreren Doppelbindungen,
- geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet

oder ausgewählt wird aus der Gruppe der Verbindungen der Formel (6),

$[C(NR^8R^9)(NR^{10}R^{11})(NR^{12}R^{13})]^+ [C(CN)_3]^-$ (6),

wobei
$R^8$ bis $R^{13}$ jeweils unabhängig voneinander

- H,
- ein geradkettiges oder verzweigtes Alkyl mit 1 bis 20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet

oder ausgewählt wird aus der Gruppe der Verbindungen der Formel (7),

$[HetN]^{z+} [C(CN)_3]^-$ (7),

wobei
- $HetN^{z+}$ ein heterocyclisches Kation, ausgewählt aus der Gruppe

Imidazolium

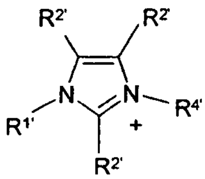

,

1H-Pyrazolium

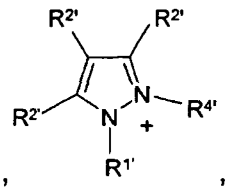

,

3H-Pyrazolium

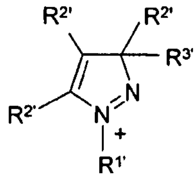

,

4H-Pyrazolium

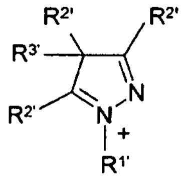

,

1-Pyrazolinium

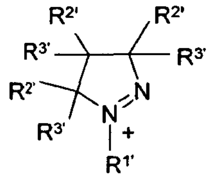

,

2-Pyrazolinium

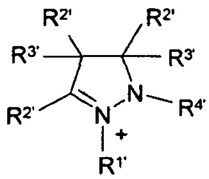

,

3-Pyrazolinium

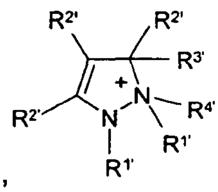

,

2,3-Dihydro-Imidazolinium

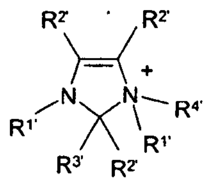

,

4,5-Dihydro-Imidazolinium

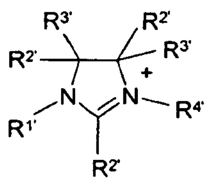

,

2,5-Dihydro-Imidazolinium

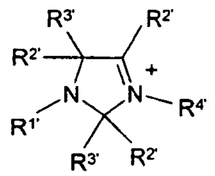

,

Pyrrolidinium

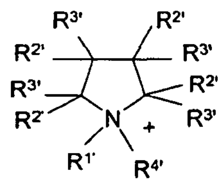

,

1,2,4-Triazolium

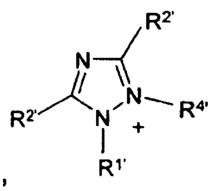

,

1,2,4-Triazolium

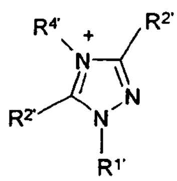

,

1,2,3-Triazolium

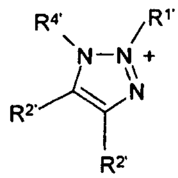

,

1,2,3-Triazolium

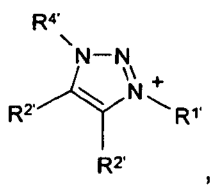

,

Pyridinium

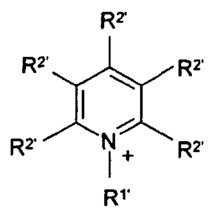

,

Pyridazinium

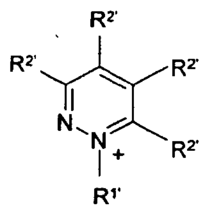

,

Pyrimidinium

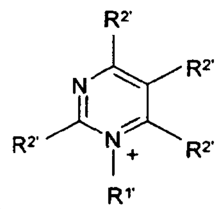

,

Piperidinium

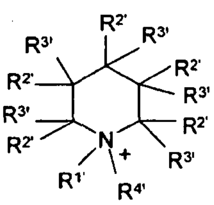

,

Morpholinium

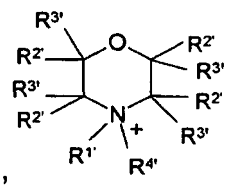

,

Piperazinium

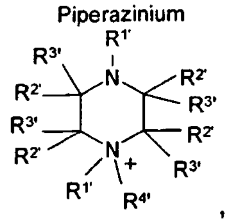

,

Piperazinium

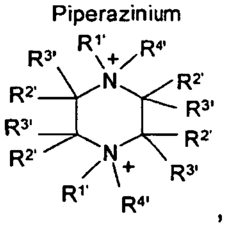

,

Pyrazinium

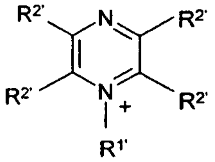

,

Thiazolium

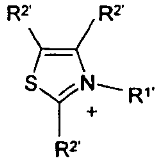

,

Oxazolium

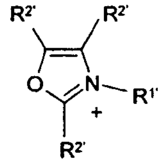

,

Indolium

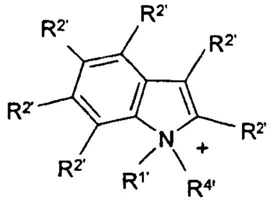

,,

Chinolinium

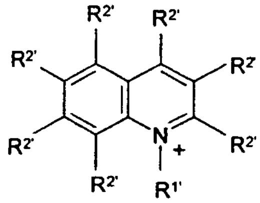

,

Isochinolinium

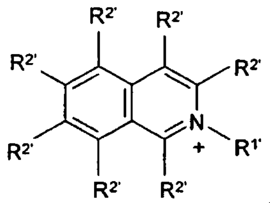

,

Chinoxalinium

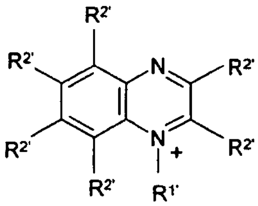

oder

Indolinium

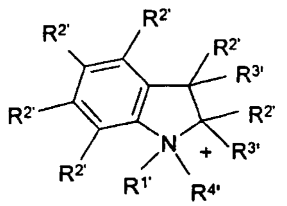

bedeutet, wobei die Substituenten
$R^{1'}$ bis $R^{4'}$ jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann,

bedeutet,
wobei die Substituenten $R^{1'}$, $R^{2'}$, $R^{3'}$ und/oder $R^{4'}$ zusammen auch ein Ringsystem bilden können.

2. Verfahren nach Anspruch 1, **dadurch gekennzeichnet, dass** das Verfahren kontinuierlich oder halbkontinuierlich durchgeführt wird.

3. Verfahren nach Anspruch 1 oder 2, **dadurch gekennzeichnet, dass** der mindestens eine Alkohol aus der Gruppe Ethanol, Isopropanol, Propanol, n-Butanol oder Isomere des n-Butanol oder Mischungen derselben gewählt wird.

4. Verfahren nach Anspruch 3, **dadurch gekennzeichnet, dass** der mindestens eine Alkohol n-Butanol ist.

5. Verfahren nach einem oder mehreren der Ansprüche 1 bis 4, **dadurch gekennzeichnet, dass** die wässrige Lösung enthaltend mindestens einen Alkohol eine Fermentationsbrühe ist.

6. Verfahren nach einem oder mehreren der Ansprüche 1 bis 5, **dadurch gekennzeichnet, dass** die Fermentationsbrühe aus einer Aceton-Butanol-Ethanol-Fermentation stammt.

**7.** Verfahren nach einem oder mehreren der Ansprüche 1 bis 6, **dadurch gekennzeichnet, dass** die mindestens eine Ionische Flüssigkeit enthaltend Tricyanomethidanionen ausgewählt wird aus der Gruppe der Verbindungen der Formeln (1) oder (2) oder der Verbindungen der Formel (7) gemäß Anspruch 1.

**8.** Verfahren nach einem oder mehreren der Ansprüche 1 bis 7, **dadurch gekennzeichnet, dass** die mindestens eine Ionische Flüssigkeit enthaltend Tricyanomethidanionen ausgewählt wird aus der Gruppe
1-Octyl-3-methylimidazolium Tricyanomethid,
1-Decyl-3-methylimidazolium Tricyanomethid,
1-Dodecyl-3-methylimidazolium Tricyanomethid,
Trihexyl-tetradecyl-ammonium Tricyanomethid,
Trihexyl-tetradecyl-phosphonium Tricyanomethid,
N-Octylpyridinium Tricyanomethid,
1-Octyl-1-methyl-pyrrolidinium Tricyanomethid,
N-Octyl-N-methyl-morpholinium Tricyanomethid,
1-Octyl-1-methyl-piperidinium Tricyanomethid.

## Claims

**1.** Method for the liquid-liquid extraction of alcohols from aqueous solutions, where

a) the aqueous solution comprising at least one alcohol is provided,
b) the aqueous solution from a) is mixed intensively with the at least one ionic liquid containing tricyanomethide anions, so that the ionic liquid is able to extract at least some of the alcohol from the aqueous solution and form an at least single-phase mixture with this alcohol,
c) the at least single-phase mixture from b) is separated off from the aqueous solution,
d) the single-phase mixture from b) is separated into the components alcohol and ionic liquid, and optionally
e) the ionic liquid from d) is fed back into step b), and where the at least one ionic liquid containing tricyanomethide anions is selected from the group of the compounds of the formula (1), (2) or (3),

$$[NR_4]^+ [C(CN)_3]^- \qquad (1),$$

$$[PR_4]^+ [C(CN)_3]^- \qquad (2),$$

$$[SR_3]^+ [C(CN)_3]^- \qquad (3),$$

where
R in each case, independently of one another, denotes

- a straight-chain or branched alkyl having 1-20 C atoms,
- a straight-chain or branched alkenyl having 2-20 C atoms and one or more double bonds,
- a straight-chain or branched alkynyl having 2-20 C atoms and one or more triple bonds,
- saturated, partially or fully unsaturated cycloalkyl having 5-7 C atoms, which may be substituted by alkyl groups having 1-6 C atoms,

with the proviso that at least two substituents R have at least 5 C atoms, or is selected from the group of the compounds of the formula (4) or (5),

$$[C(NR^3R^4)(OR^5)(NR^6R^7)]^+ [C(CN)_3]^- \qquad (4),$$

$$[C(NR^3R^4)(SR^5)(NR^6R^7)]^+ [C(CN)_3]^- \qquad (5),$$

where
$R^3$ to $R^7$ each, independently of one another, denote

- H, where H is excluded for $R^5$,

- a straight-chain or branched alkyl having 1 to 20 C atoms,
- a straight-chain or branched alkenyl having 2-20 C atoms and one or more double bonds,
- a straight-chain or branched alkynyl having 2-20 C atoms and one or more triple bonds,
- saturated, partially or fully unsaturated cycloalkyl having 3-7 C atoms, which may be substituted by alkyl groups having 1-6 C atoms,

or is selected from the group of the compounds of the formula (6),

$$[C(NR^8R^9)(NR^{10}R^{11})(NR^{12}R^{13})]^+ \; [C(CN)_3]^- \qquad (6),$$

where
$R^8$ to $R^{13}$ each, independently of one another, denote

- H,
- a straight-chain or branched alkyl having 1 to 20 C atoms,
- a straight-chain or branched alkenyl having 2-20 C atoms and one or more double bonds,
- a straight-chain or branched alkynyl having 2-20 C atoms and one or more triple bonds,
- saturated, partially or fully unsaturated cycloalkyl having 3-7 C atoms, which may be substituted by alkyl groups having 1-6 C atoms,

or is selected from the group of the compounds of the formula (7),

$$[HetN]^{z+} \; [C(CN)_3]^- \qquad (7),$$

where

- $HetN^{z+}$ denotes a heterocyclic cation selected from the group

imidazolium
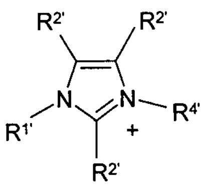
,
1H-pyrazolium
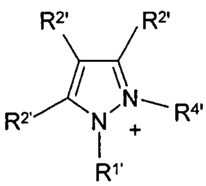
,
3H-pyrazolium
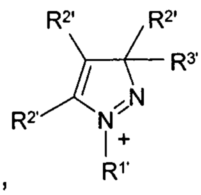
,
4H-pyrazolium
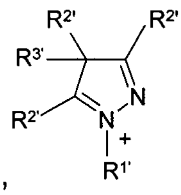
,
1-pyrazolinium
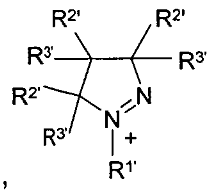
,

2-pyrazolinium
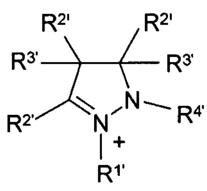
,
3-pyrazolinium
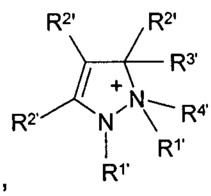
,
2,3-dihydroimidazolinium
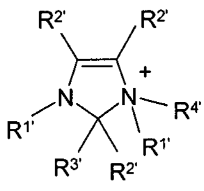
,
4,5-dihydroimidazolinium
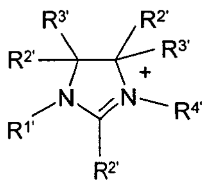
,

2,5-dihydroimidazolinium
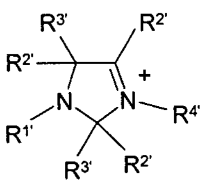
,
pyrrolidinium
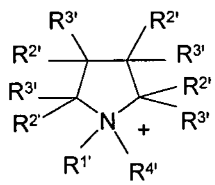
,
1,2,4-triazolium
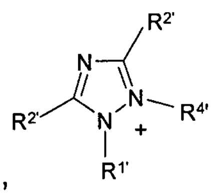
,
1,2,4-triazolium
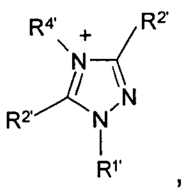
,

1,2,3-triazolium

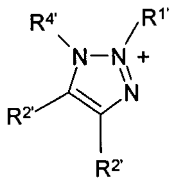

,

1,2,3-triazolium

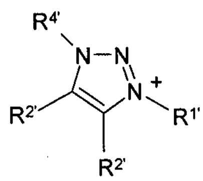

,

pyridinium

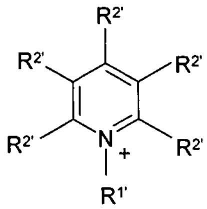

,

pyridazinium

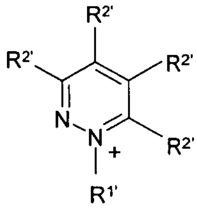

,

pyrimidinium

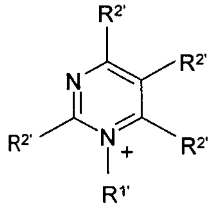

,

piperidinium

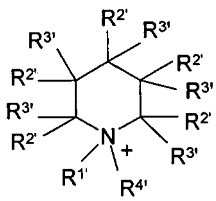

,

morpholinium

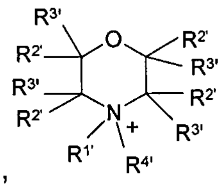

,

piperazinium

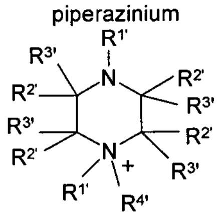

,

piperazinium

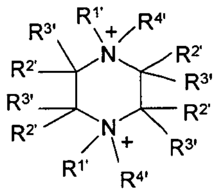

,

pyrazinium

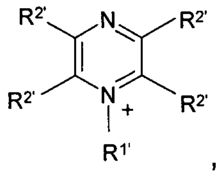

,

thiazolium

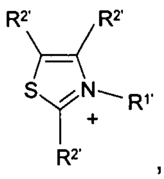

,

oxazolium

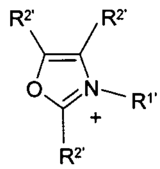

,

indolium

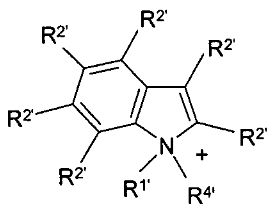

,

quinolinium

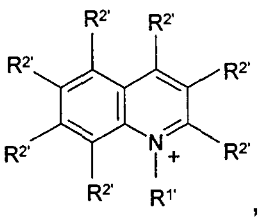

,

isoquinolinium

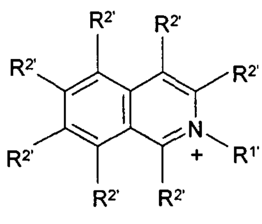

,

quinoxalinium

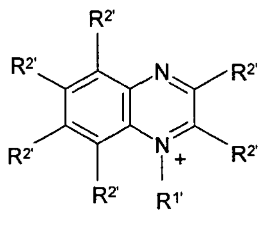

or

indolinium

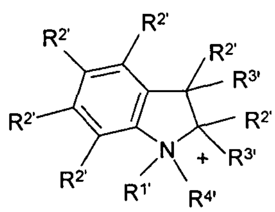

where the substituents
$R^{1'}$ to $R^{4'}$ each, independently of one another, denote

- a straight-chain or branched alkyl having 1-20 C atoms,
- a straight-chain or branched alkenyl having 2-20 C atoms and one or more double bonds,

- a straight-chain or branched alkynyl having 2-20 C atoms and one or more triple bonds,
- saturated, partially or fully unsaturated cycloalkyl having 5-7 C atoms, which may be substituted by alkyl groups having 1-6 C atoms,

where the substituents $R^{1'}$, $R^{2'}$, $R^{3'}$ and/or $R^{4'}$ together may also form a ring system.

2. Method according to Claim 1, **characterised in that** the method is carried out continuously or semi-continuously.

3. Method according to Claim 1 or 2, **characterised in that** the at least one alcohol is selected from the group ethanol, isopropanol, propanol, n-butanol or isomers of n-butanol, or mixtures thereof.

4. Method according to Claim 3, **characterised in that** the at least one alcohol is n-butanol.

5. Method according to one or more of Claims 1 to 4, **characterised in that** the aqueous solution comprising at least one alcohol is a fermentation broth.

6. Method according to one or more of Claims 1 to 5, **characterised in that** the fermentation broth originates from an acetone-butanol-ethanol fermentation.

7. Method according to one or more of Claims 1 to 6, **characterised in that** the at least one ionic liquid containing tricyanomethide anions is selected from the group of the compounds of the formula (1) or (2) or the compounds of the formula (7) according to Claim 1.

8. Method according to one or more of Claims 1 to 7, **characterised in that** the at least one ionic liquid containing tricyanomethide anions is selected from the group
1-octyl-3-methylimidazolium tricyanomethide,
1-decyl-3-methylimidazolium tricyanomethide,
1-dodecyl-3-methylimidazolium tricyanomethide,
trihexyltetradecylammonium tricyanomethide,
trihexyltetradecylphosphonium tricyanomethide,
N-octylpyridinium tricyanomethide,
1-octyl-1-methylpyrrolidinium tricyanomethide,
N-octyl-N-methylmorpholinium tricyanomethide,
1-octyl-1-methylpiperidinium tricyanomethide.

## Revendications

1. Méthode d'extraction liquide-liquide d'alcools à partir de solutions aqueuses, dans laquelle

a) on fournit la solution aqueuse comprenant au moins un alcool,
b) la solution aqueuse issue de a) est mélangée vigoureusement avec le au moins un liquide ionique contenant des anions tricyanométhanure, de sorte que le liquide ionique soit capable d'extraire au moins une partie de l'alcool à partir de la solution aqueuse et former un mélange au moins à phase unique avec cet alcool,
c) le mélange au moins à phase unique issu de b) est séparé de la solution aqueuse,
d) le mélange à phase unique issu de b) est séparé en composants d'alcool et de liquide ionique, et éventuellement
e) le liquide ionique issu de d) est réalimenté dans l'étape b), et où le au moins un liquide ionique contenant des anions tricyanométhanure est choisi dans le groupe constitué par les composés de formule (1), (2) ou (3),

$$[NR_4]^+ [C(CN)_3]^- \quad (1),$$

$$[PR_4]^+ [C(CN)_3]^- \quad (2),$$

$$[SR_3]^+ [C(CN)_3]^- \quad (3),$$

où
R désigne dans chaque cas, indépendamment les uns des autres,

- alkyle à chaîne linéaire ou ramifiée ayant 1-20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 5-7 atomes de C, pouvant être substitué par des groupements alkyle ayant 1-6 atomes de C,

à condition qu'au moins deux substituants R aient au moins 5 atomes de C, ou est choisi dans le groupe constitué par les composés de formule (4) ou (5),

$$[C(NR^3R^4)(OR^5)(NR^6R^7)]^+ [C(CN)_3]^- \quad (4),$$

$$[C(NR^3R^4)(SR^5)(NR^6R^7)]^+ [C(CN)_3]^- \quad (5),$$

où
$R^3$ à $R^7$ désignent chacun, indépendamment les uns des autres,

- H, où H est exclu pour $R^5$,
- alkyle à chaîne linéaire ou ramifiée ayant 1 à 20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 3-7 atomes de C, pouvant être substitué par des groupements alkyle ayant 1-6 atomes de C,

ou est choisi dans le groupe constitué par les composés de formule (6),

$$[C(NR^8R^9)(NR^{10}R^{11})(NR^{12}R^{13})]^+ [C(CN)_3]^- \quad (6),$$

où
$R^8$ à $R^{13}$ désignent chacun, indépendamment les uns des autres,

- H,
- alkyle à chaîne linéaire ou ramifiée ayant 1 à 20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 3-7 atomes de C, pouvant être substitué par des groupements alkyle ayant 1-6 atomes de C,

ou est choisi dans le groupe constitué par les composés de formule (7),

$$[HétN]^{z+} [C(CN)_3]^- \quad (7),$$

où

- HétN$^{z+}$ désigne un cation hétérocyclique choisi dans le groupe constitué par

imidazolium

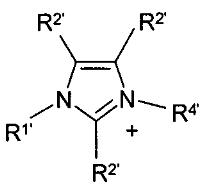

,

1H-pyrazolium

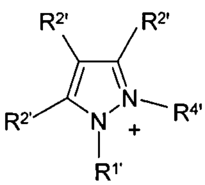

,

3H-pyrazolium

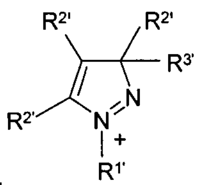

,

4H-pyrazolium

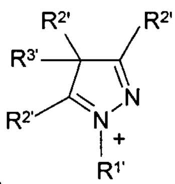

,

1-pyrazolinium

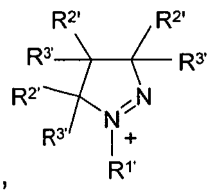

,

2-pyrazolinium

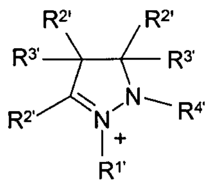

,

3-pyrazolinium

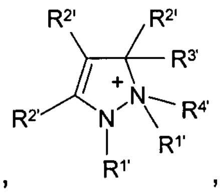

,

2,3-dihydroimidazolinium

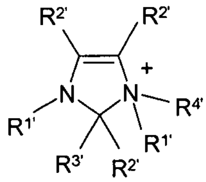

,

4,5-dihydroimidazolinium

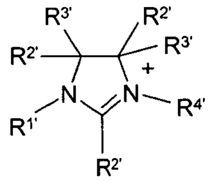

,

2,5-dihydroimidazolinium

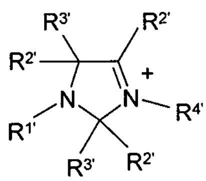

,

pyrrolidinium

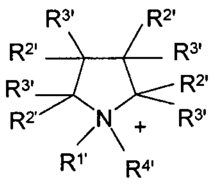

,

1,2,4-triazolium

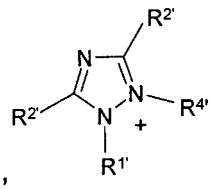

,

1,2,4-triazolium

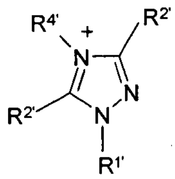

,

1,2,3-triazolium

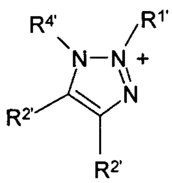

,

1,2,3-triazolium

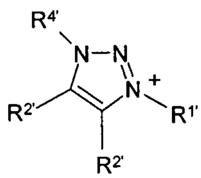

,

pyridinium

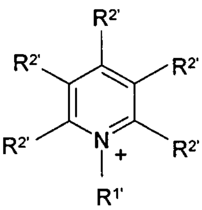

,

pyridazinium

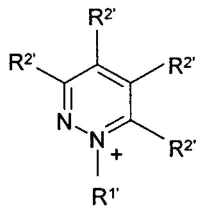

,

pyrimidinium

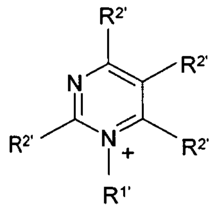

,

pipéridinium

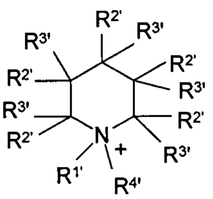

,

morpholinium

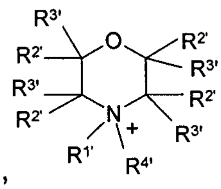

,

pipérazinium

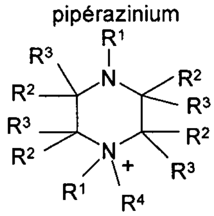

,

pipérazinium

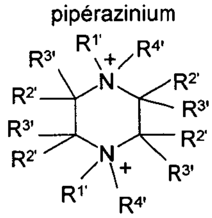

,

pyrazinium

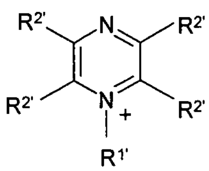

,

thiazolium

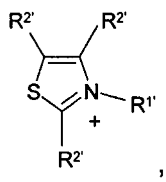

,

oxazolium

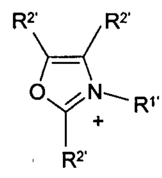

,

indolium

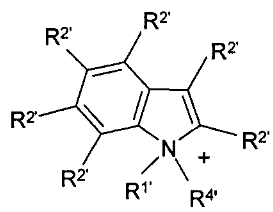

,

quinoléinium

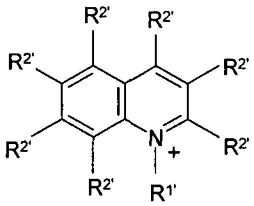

,

isoquinoléinium

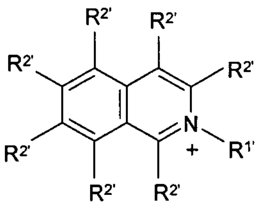

,

quinoxalinium

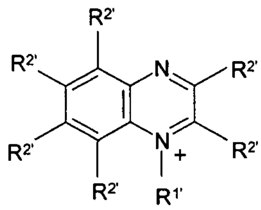

ou

indolinium

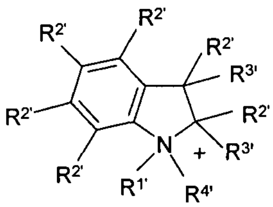

où les substituants
$R^{1'}$ à $R^{4'}$ désignent chacun, indépendamment les uns des autres,

- alkyle à chaîne linéaire ou ramifiée ayant 1-20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 5-7 atomes de C, pouvant être substitué par des groupements alkyle ayant 1-6 atomes de C,

où les substituants $R^{1'}$, $R^{2'}$, $R^{3'}$ et/ou $R^{4'}$ peuvent aussi former ensemble un noyau.

2. Méthode selon la revendication 1, **caractérisée en ce que** la méthode est mise en oeuvre de manière continue ou semi-continue.

3. Méthode selon la revendication 1 ou 2, **caractérisée en ce que** le au moins un alcool est choisi dans le groupe constitué par l'éthanol, l'isopropanol, le propanol, le n-butanol ou des isomères du n-butanol, ou des mélanges de ceux-ci.

4. Méthode selon la revendication 3, **caractérisée en ce que** le au moins un alcool est le n-butanol.

5. Méthode selon l'une ou plusieurs parmi les revendications 1 à 4, **caractérisée en ce que** la solution aqueuse comprenant au moins un alcool est un bouillon de fermentation.

6. Méthode selon l'une ou plusieurs parmi les revendications 1 à 5, **caractérisée en ce que** le bouillon de fermentation est issu d'une fermentation acétone-butanol-éthanol.

7. Méthode selon l'une ou plusieurs parmi les revendications 1 à 6, **caractérisée en ce que** le au moins un liquide ionique contenant des anions tricyanométhanure est choisi dans le groupe constitué par les composés de formule (1) ou (2) ou les composés de formule (7) selon la revendication 1.

8. Méthode selon l'une ou plusieurs parmi les revendications 1 à 7, **caractérisée en ce que** le au moins un liquide ionique contenant des anions tricyanométhanure est choisi dans le groupe constitué par
le tricyanométhanure de 1-octyl-3-méthylimidazolium,
le tricyanométhanure de 1-décyl-3-méthylimidazolium,
le tricyanométhanure de 1-dodécyl-3-méthylimidazolium,
le tricyanométhanure de trihexyltétradécylammonium,

le tricyanométhanure de trihexyltétradécylphosphonium,
le tricyanométhanure de N-octylpyridinium,
le tricyanométhanure de 1-octyl-1-méthylpyrrolidinium,
le tricyanométhanure de N-octyl-N-méthylmorpholinium,
le tricyanométhanure de 1-octyl-1-méthylpipéridinium.

Fig. 1

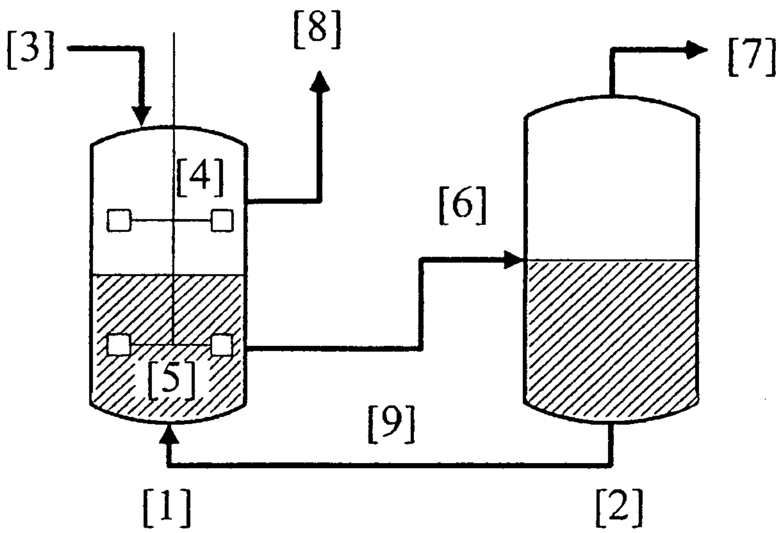

Fig. 2

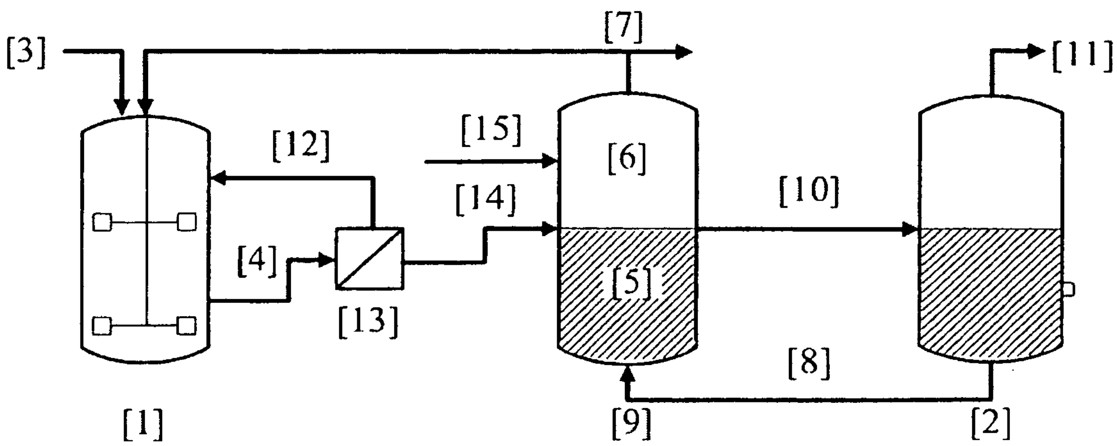

Fig. 3

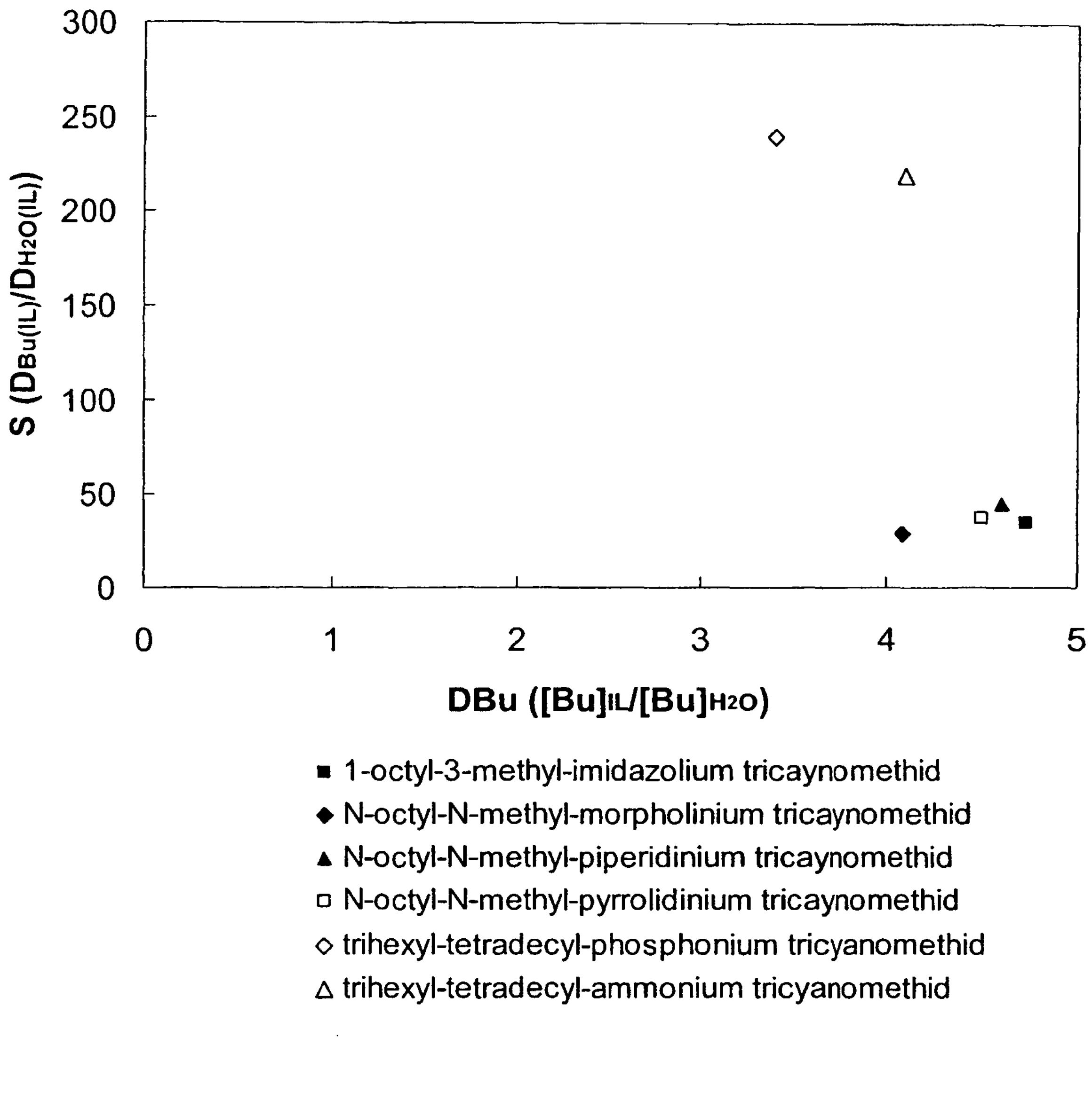
300
250
200
150
100
50
0
S (DBu(IL)/DH2O(IL))
0
1
2
3
4
5
DBu ([Bu]IL/[Bu]H2O)
■ 1-octyl-3-methyl-imidazolium tricaynomethid
◆ N-octyl-N-methyl-morpholinium tricaynomethid
▲ N-octyl-N-methyl-piperidinium tricaynomethid
□ N-octyl-N-methyl-pyrrolidinium tricaynomethid
◇ trihexyl-tetradecyl-phosphonium tricyanomethid
△ trihexyl-tetradecyl-ammonium tricyanomethid

Fig. 4

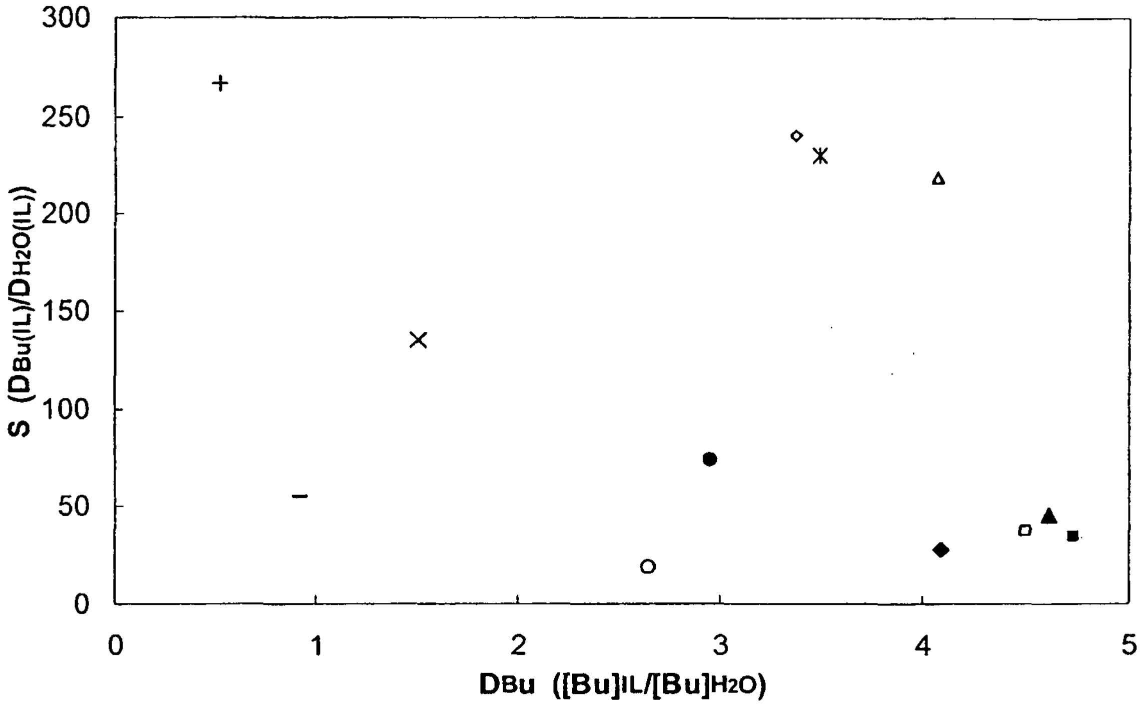


+ 1-Octyl-3-methylimidazolium tris(pentafluoroethyl)trifluorophosphat
− 1-Octyl-3-methylimidazolium hexafluorophosphat
× 1-Octyl-3-methylimidazolium bis(trifluoromethyl)sulfonylimid
○ 1-Octyl-3-methylimidazolium tetrafluoroborat
● 1-Octyl-3-methylimidazolium tetracyanoborat
■ 1-Octyl-3-methylimidazolium tricyanomethid
□ 1-Octyl-1-methylpyrrolidinium tricyanomethid
▲ 1-Octyl-1-methylpiperidinium tricyanomethid
◆ 1-Octyl-1-methylmorpholinium tricyanomethid
◇ Trihexyl(tetradecyl)phosphonium tricyanomethid
△ Trihexyl(tetradecyl)ammonium tricyanomethid
⁎ Oleyl Alcohol

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006021390 A


### In der Beschreibung aufgeführte Nicht-Patentliteratur


- **J.F. BRENNECKE et al.** *J. Chem. Eng. Data,* 2007, vol. 52, 2462-2467
- **A.G. FADEEV.** *Chem. Commun.,* 2001, 295-296
- **J. RYDBERG ; M. COX ; C. MUSKAS ; G.R. CHOPPPIN.** *Solvent Extraction Principles and Practice,* 2004
- **R.H. PERRY.** Perry's chemical engineer's handbook. 1984